# EUROPÄISCHE PATENTSCHRIFT

(11) **EP 0 796 846 B1**
(45) Veröffentlichungstag und Bekanntmachung des Hinweises auf die Patenterteilung: **19.07.2000**
(21) Anmeldenummer: 97103813.8
(22) Anmeldetag: 07.03.1997
(51) Int. Cl.: C07D 213/30, C07D 213/50, C07D 213/38, C07D 401/06, C07D 417/06, A61K 31/44

(54) **2-Aryl-substituierte Pyridine**
2-Aryl substituted pyridines
Pyridines substituées en position 2 par un reste aryle

(30) Priorität: 20.03.1996 DE 19610932
(43) Veröffentlichungstag der Anmeldung: 24.09.1997
(73) Patentinhaber: BAYER AG, 51368 Leverkusen (DE)
(72) Erfinder: Schmidt, Gunter, Dr., 42115 Wuppertal (DE); Angerbauer, Rolf, Dr., Higashinada-ku, Kobe-shi (JP); Brandes, Arndt, Dr., 42115 Wuppertal (DE); Lögers, Michael, Dr., 42327 Wuppertal (DE); Müller-Gliemann, Matthias, Dr., 42719 Solingen (DE); Bischoff, Hilmar, Dr., 42113 Wuppertal (DE); Schmidt, Delf, Dr., 42113 Wuppertal (DE); Wohlfeil, Stefan, Dr., 40721 Hilden (DE)

(56) Entgegenhaltungen:
- EP-A- 0 306 929
- EP-A- 0 307 342
- EP-A- 0 325 130
- EP-A- 0 356 788
- EP-A- 0 603 699

## Beschreibung

Die vorliegende Erfindung betrifft 2-Aryl-substituierte Pyridine, Verfahren zu ihrer Herstellung und ihre Verwendung in Arzneimitteln.

Aus der US 5 169 857 sind 7-(polysubstituierte Pyridyl) 6-heptenoate zur Behandlung der Arteriosklerose, Lipoproteinaemia und Hyperproteinaemia bekannt. Außerdem wird die Herstellung von 7-(4-Aryl-3-pyridyl)-3,5-dihydroxy-6-heptenoate in der EP 325 130 beschrieben.

Die vorliegende Erfindung betrifft 2-Aryl-substituierte Pyridine der allgemeinen Formel (I), 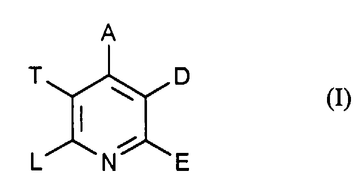 in welcher
- A und E: gleich oder verschieden sind und für Aryl mit 6 bis 10 Kohlenstoffatomen stehen, das gegebenenfalls bis zu 5-fach gleich oder verschieden durch Halogen, Hydroxy, Trifluormethyl, Trifluormethoxy, Nitro oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR¹R² substituiert ist,
worin
R¹ und R² gleich oder verschieden sind und Wasserstoff, Phenyl oder geradkettiges oder verzweigtes Alkyl mit bis zu 6 Kohlenstoffatomen bedeuten,
- D: für geradkettiges oder verzweigtes Alkyl mit bis zu 8 Kohlenstoffatomen steht, das durch Hydroxy substituiert ist,
- L: für Cycloalkyl mit 3 bis 8 Kohlenstoffatomen oder für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
- T: für einen Rest der Formel

R³―X―

oder 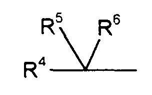 steht,
worin
R³ und R⁴ gleich oder verschieden sind und Cycloalkyl mit 3 bis 8 Kohlenstoffatomen bedeuten, oder
Aryl mit 6 bis 10 Kohlenstoffatomen, oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 3-fach gleich oder verschieden durch Trifluormethyl, Trifluormethoxy, Nitro, Halogen, Hydroxy, Carboxyl, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Phenylthio substituiert sind, die ihrerseits durch Halogen, Trifluormethyl oder Trifluormethoxy substituiert sein können,
und/oder die Cyclen gegebenenfalls durch eine Gruppe der Formel -NR⁷R⁸ substituiert sind,
worin
R⁷ und R⁸ gleich oder verschieden sind und die oben angegebene Bedeutung von R¹ und R² haben,
X geradkettiges oder verzweigtes Alkyl oder Alkenyl mit jeweils 2 bis zu 10 Kohlenstoffatomen bedeutet, die gegebenenfalls bis zu 2-fach durch Hydroxy oder Halogen substituiert sind,
R⁵ Wasserstoff bedeutet, und und
R⁶ Wasserstoff, Halogen, Azido, Trifluormethyl, Mercapto, Hydroxy, Trifluormethoxy, geradkettiges oder verzweigtes Alkoxy mit bis zu 5 Kohlenstoffatomen oder einen Rest der Formel -NR⁹R¹⁰ bedeutet, worin
R⁹ und R¹⁰ gleich oder verschieden sind und die oben angegebene Bedeutung von R¹ und R² haben, oder
R⁵ und R⁶ gemeinsam mit dem Kohlenstoffatom eine Carbonylgruppe bilden,
und deren Salze.

Die erfindungsgemäßen 2-Aryl-substituierten Pyridine können auch in Form ihrer Salze vorliegen. Im allgemeinen seien hier Salze mit organischen oder anorganischen Basen oder Säuren genannt.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen, wie beispielsweise Ethylamin, Di- bzw. Triethylamin, Di- bzw. Triethanolamin, Dicyclohexylamin, Dimethylaminoethanol, Arginin, Lysin, Ethylendiamin oder 2-Phenylethylamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren als auch deren jeweiligen Mischungen. Diese Mischungen der Enantiomeren und Diastereomeren lassen sich in bekannter Weise in die stereoisomer einheitlichen Bestandteile trennen.

Heterocyclus, gegebenenfalls benzokondensiert, steht im Rahmen der Erfindung im allgemeinen für einen gesättigten oder ungesättigten 5- bis 7-gliedrigen, vorzugsweise 5- bis 6-gliedrigen Heterocyclus, der bis zu 3 Heteroatome aus der Reihe S, N und/oder O enthalten kann. Beispielsweise seien genannt: Indolyl, Isochinolyl, Chinolyl, Benzo[b]thiophen, Benzothiazolyl, Benzo[b]furanyl, Pyridyl, Thienyl, Furyl, Pyrrolyl, Thiazolyl, Oxazolyl, Imidazolyl, Morpholinyl oder Piperidyl. Bevorzugt sind Chinolyl, Indolyl, Pyridyl und Benzothiazolyl.

Bevorzugt sind Verbindungen der allgemeinen Formel (I),
in welcher
- A und E: gleich oder verschieden sind und für Phenyl oder Naphthyl stehen, die gegebenenfalls bis zu 2-fach gleich oder verschieden durch Fluor, Chlor, Brom, Hydroxy, Trifluormethyl, Trifluormethoxy, Nitro oder durch geradkettiges oder verzweigtes Alkyl, Acyl oder Alkoxy mit jeweils bis zu 6 Kohlenstoffatomen oder durch eine Gruppe der Formel -NR¹R² substituiert sind,
worin
R¹ und R² gleich oder verschieden sind und Wasserstoff, Phenyl oder geradkettiges oder verzweigtes Alkyl mit bis zu 4 Kohlenstoffatomen bedeuten,
- D: für geradkettiges oder verzweigtes Alkyl mit bis zu 7 Kohlenstoffatomen steht, das durch Hydroxy substituiert ist,
- L: für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
- T: für einen Rest der Formel

R³―X―

oder 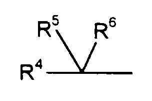 steht,
worin
R³ und R⁴ gleich oder verschieden sind und Cyclopropyl, Cyclopentyl oder Cyclohexyl bedeuten, oder Napthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Phenylthio substituiert sind, die ihrerseits durch Fluor, Chlor, Brom, Trifluormethyl oder Trifluormethoxy substituiert sein können,
und/oder die Cyclen gegebenenfalls durch eine Gruppe der Formel -NR⁷R⁸ substituiert sind,
worin
R⁷ und R⁸ gleich oder verschieden sind und die oben angegebene Bedeutung von R¹ und R² haben,
X geradkettiges oder verzweigtes Alkyl oder Alkenyl mit jeweils 2 bis zu 8 Kohlenstoffatomen bedeutet, die gegebenenfalls bis zu 2-fach durch Hydroxy oder Fluor substituiert sind,
R⁵ Wasserstoff bedeutet,
und
R⁶ Wasserstoff, Fluor, Chlor, Brom, Azido, Trifluormethyl, Hydroxy, Mercapto, Trifluormethoxy, geradkettiges oder verzweigtes Alkoxy mit bis zu 4 Kohlenstoffatomen oder einen Rest der Formel -NR⁹R¹⁰ bedeutet,
worin
R⁹ und R¹⁰ gleich oder verschieden sind und die oben angegebene Bedeutung von R¹ und R² haben, oder
R⁵ und R⁶ gemeinsam mit dem Kohlenstoffatom eine Carbonylgruppe bilden,
und deren Salze.

Besonders bevorzugt sind Verbindungen der allgemeinen Formel (I),
in welcher
- A und E: gleich oder verschieden sind und für Phenyl oder Naphthyl stehen, die gegebenenfalls bis zu 2-fach gleich oder verschieden durch Fluor, Chlor, Brom, Hydroxy, Trifluormethyl, Trifluormethoxy, Nitro oder durch geradkettiges oder verzweigtes Alkyl oder Alkoxy mit jeweils bis zu 5 Kohlenstoffatomen substituiert sind,
- D: für geradkettiges oder verzweigtes Alkyl mit bis zu 6 Kohlenstoffatomen steht, das durch Hydroxy substituiert ist,
- L: für Cyclopropyl, Cyclobutyl, Cyclopentyl, Cyclohexyl oder Cycloheptyl steht, oder
für geradkettiges oder verzweigtes Alkyl mit bis zu 5 Kohlenstoffatomen steht, das gegebenenfalls durch Cyclopentyl oder Cyclohexyl substituiert ist,
- T: für einen Rest der Formel

R³―X―

oder 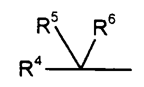 steht,
worin
R³ und R⁴ gleich oder verschieden sind und Cyclopropyl, Phenyl, Pyridyl, Chinolyl, Indolyl, Naphthyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, Amino, durch geradkettiges oder verzweigtes Alkyl, Alkoxy oder Alkoxycarbonyl mit jeweils bis zu 4 Kohlenstoffatomen oder durch Phenyl, Phenoxy oder Phenylthio substituiert sind, die ihrerseits durch Fluor, Chlor, Brom, Trifluormethyl oder Trifluormethoxy substituiert sein können,
worin
X geradkettiges oder verzweigtes Alkyl oder Alkenyl mit 2 bis zu 6 Kohlenstoffatomen bedeutet, die gegebenenfalls bis zu 2-fach durch Hydroxy oder Fluor substituiert sind,
R⁵ Wasserstoff bedeutet, und
R⁶ Wasserstoff, Fluor, Chlor, Brom, Azido, Trifluormethyl, Amino, Hydroxy, Trifluormethoxy, Methoxy oder Mercapto bedeutet, oder
R⁵ und R⁶ gemeinsam mit dem Kohlenstoffatom eine Carbonylgruppe bilden,
und deren Salze.

Ganz besonders bevorzugt sind erfindungsgemäße Verbindungen der allgemeinen Formel (I),
in welcher
- A: für Phenyl steht, das gegebenenfalls bis zu 2-fach gleich oder verschieden durch Fluor, Chlor, Methyl, Nitro oder Methoxy substituiert ist.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (II) oder (III) 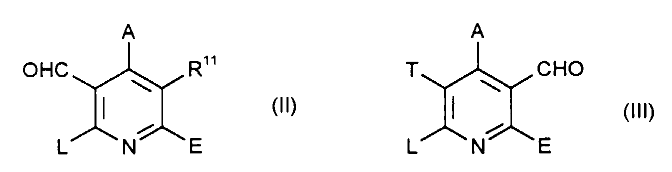 in welcher
- A, E, L und T: die oben angegebene Bedeutung haben,
und
- R¹¹: für geradkettiges oder verzweigtes Alkoxycarbonyl mit bis zu 4 Kohlenstoffatomen steht,
entweder zunächst mit metallorganischen Reagenzien, insbesondere Grignard- oder Wittig-Reaktionen in inerten Lösemitteln umsetzt, gegebenenfalls weitere Derivatisierung nach üblichen Methoden durchführt und anschließend in inerten Lösemitteln reduziert,
oder im Fall der Verbindungen der allgemeinen Formel (III) direkt Reduktionen, gegebenenfalls über mehrere Stufen, durchführt.

Die erfindungsgemäßen Verfahren können durch folgendes Formelschema beispielhaft erläutert werden:

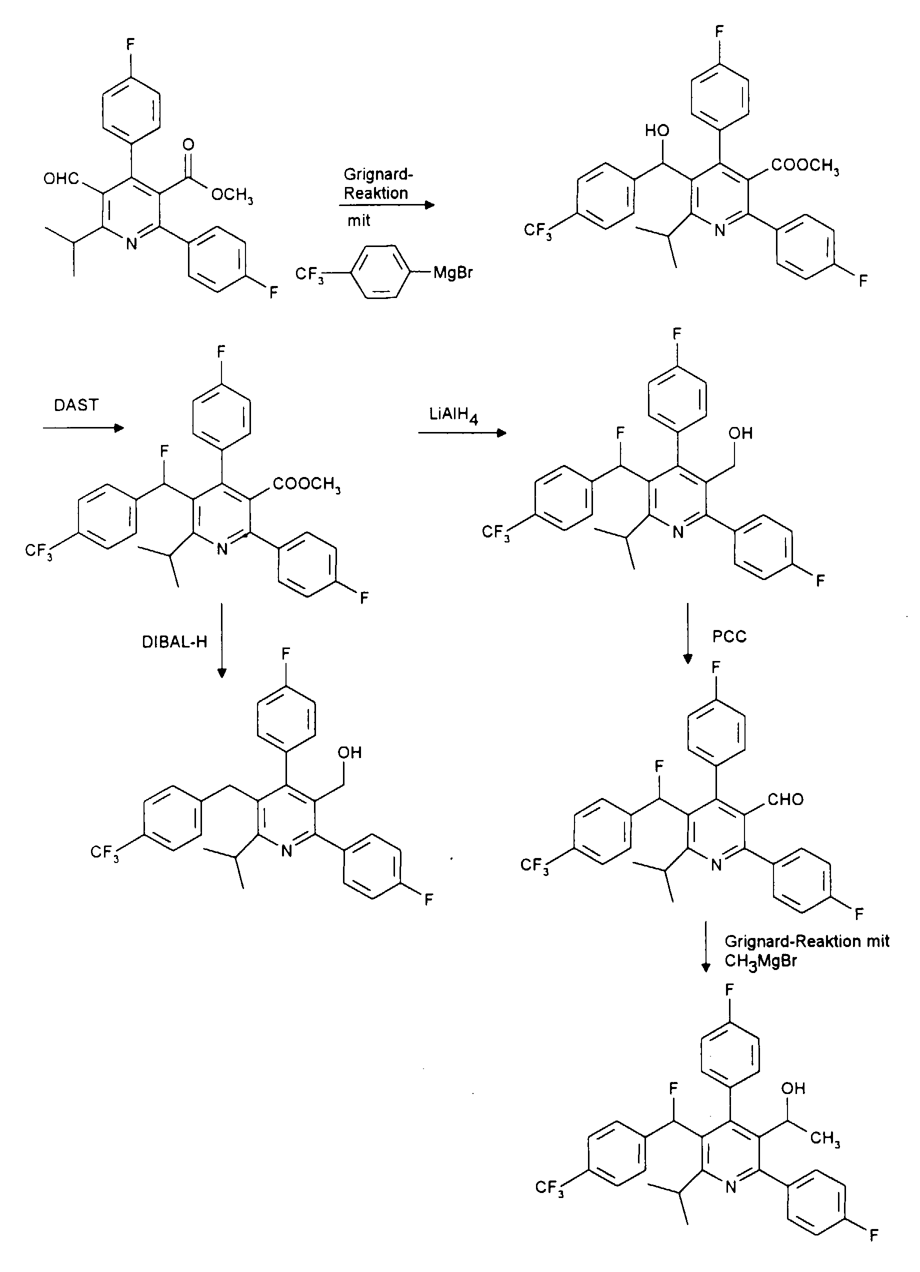

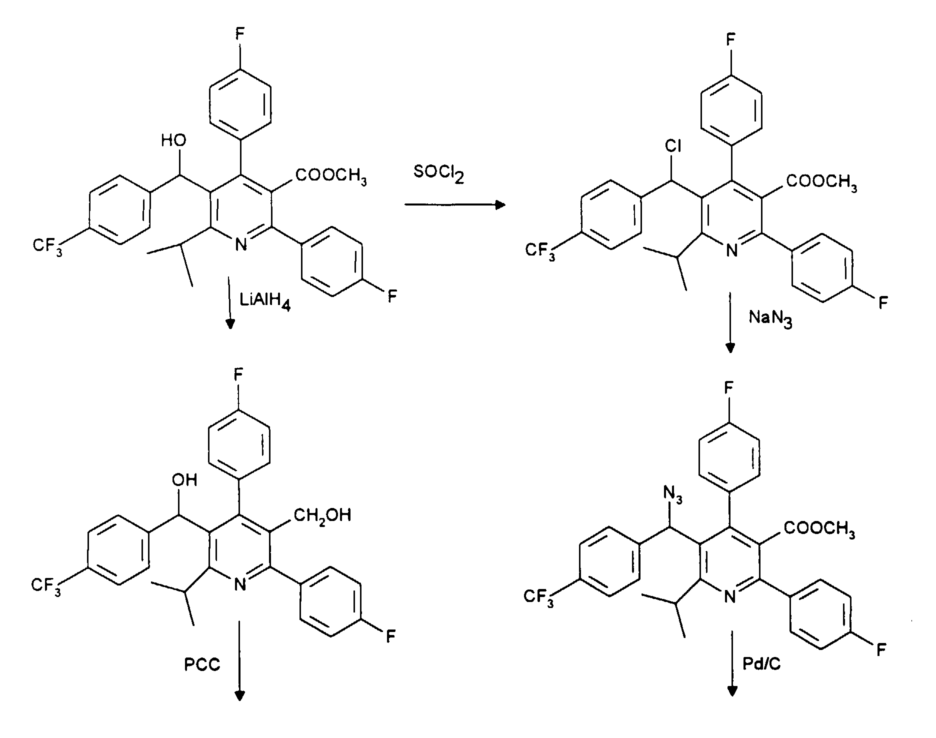

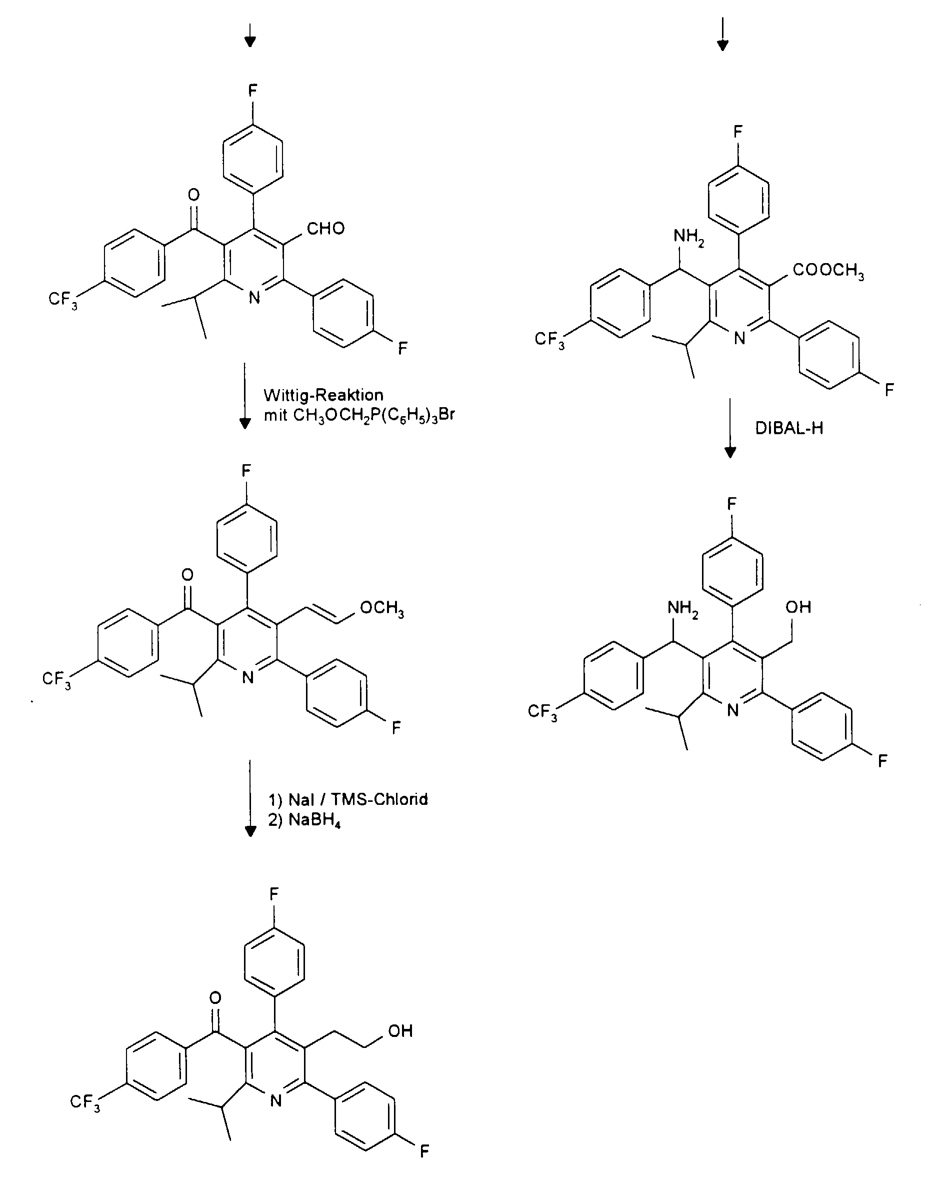

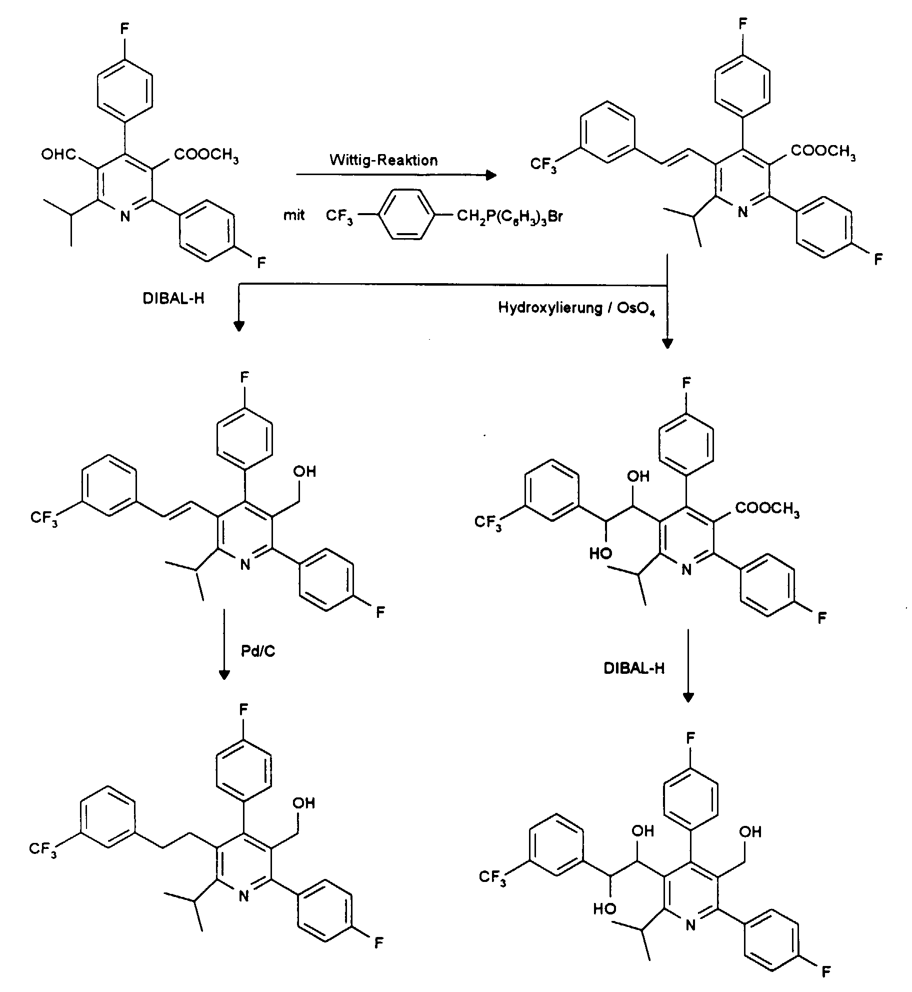

Als Lösemittel eignen sich Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als metallorganische Reagenzien eignen sich beispielsweise Grignard-Systeme wie Mg/Brombenzotrifluorid und p-Trifluormethylphenyllithium. Bevorzugt ist das System Mg/Brombenzotrifluorid.

Die Reduktionen und die Derivatisierungen erfolgen nach den oben aufgeführten Methoden.

Die Reduktionen erfolgen im allgemeinen in Ethern, wie beispielsweise Dioxan, Tetrahydrofuran oder Diethylether oder in Kohlenwasserstoffen wie beispielsweise Benzol, Hexan oder Toluol. Bevorzugt sind Toluol und Tetrahydrofuran.

Als Reduktionsmittel eignen sich komplexe Metallhydride, wie beispielsweise Lithiumaluminiumhydrid, Natriumcyanoborhydrid, Natriumaluminiumhydrid, Diisobutylaluminiumhydrid, Dimethoxymethylaluminat-Natriumsalz oder Natrium-bis-(2-methoxyethoxy)dihydroaluminat (Red-Al). Bevorzugt werden Diisobutylaluminiumhydrid (DIBAL-H) und Dimethoxymethylaluminat- Natriumsalz.

Das Reduktionsmittel wird im allgemeinen in einer Menge von 4 mol bis 10 mol, bevorzugt von 4 mol bis 5 mol bezogen auf 1 mol der zu reduzierenden Verbindungen eingesetzt.

Die Reduktion verläuft im allgemeinen in einem Temperaturbereich von -78°C bis +50°C, bevorzugt von -78°C bis 0°C, besonders bevorzugt bei -78°C, jeweils in Abhängigkeit von der Wahl des Reduktionsmittels sowie Lösemittel.

Die Reduktion verläuft im allgemeinen bei Normaldurck, es ist aber auch möglich bei erhöhtem oder erniedrigtem Druck zu arbeiten.

Die Reduktionen können aber auch mit Reduktionsmitteln, die für die Reduktion von Ketonen zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat oder Lithiumaluminiumhydrid durchgeführt. Ganz besonders bevorzugt wird die Reduktion mit Natriumborhydrid, in Anwesenheit von Triethylboran durchgeführt.

Die Hydrierung erfolgt nach üblichen Methoden mit Wasserstoff in Anwesenheit von Edelmetalkatalysatoren, wie beispielsweise Pd/C, Pt/C oder Raney-Nickel in einem der oben aufgeführten Lösemittel, vorzugsweise in Alkoholen wie beispielsweise Methanol, Ethanol oder Propanol, in einem Temperaturbereich von -20°C bis +100°C, bevorzugt von 0°C bis +50°C, bei Normaldruck oder Überdruck.

Als Derivatisierungen seien beispielhaft folgende Reaktionstypen genannt: Oxidationen, Reduktionen, Hydrierungen, Halogenierung, Wittig/Grignard-Reaktionen und Amidierungen/Sulfoamidierungen.

Als Basen kommen für die einzelnen Schritte die üblichen stark basischen Verbindungen in Frage. Hierzu gehören bevorzugt lithiumorganische Verbindungen wie beispielsweise N-Butyllithium, sec.-Butyllithium, tert.Butyllithium oder Phenyllithium, oder Amide wie beispielsweise Lithiumdiisopropylamid, Natriumamid oder Kaliumamid, oder Lithiumhexamethylsilylamid, oder Alkalihydride wie Natriumhydrid oder Kaliumhydrid. Besonders bevorzugt werden N-Butyllithium und Natriumhydrid eingesetzt.

Als Basen eignen sich außerdem die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat. Besonders bevorzugt werden Natriumhydroxid oder Kaliumhydroxid eingesetzt.

Als Lösemittel eignen sich für die einzelnen Reaktionsschritte auch Alkohole wie Methanol, Ethanol, Propanol, Butanol oder tert.Butanol. Bevorzugt ist tert.Butanol.

Gegebenenfalls ist es nötig, einige Reaktionsschritte unter Schutzgasatmosphäre durchzuführen.

Die Halogenierungen erfolgen im allgemeinen in einem der oben aufgeführten chlorierten Kohlenwasserstoffen, wobei Methylenchlorid bevorzugt ist.

Als Halogenierungsmittel eignen sich beispielsweise Diethylamino-Schwefeltrifluorid (DAST) oder SOCl₂.

Die Halogenierung verläuft im allgemeinen in einem Temperaturbereich von -78°C bis +50°C, bevorzugt von -78°C bis 0°C, besonders bevorzugt bei -78°C, jeweils in Abhängigkeit von der Wahl des Halogenierungsmittels sowie Lösemittel.

Die Halogenierung verläuft im allgemeinen bei Normaldurck, es ist aber auch möglich bei erhöhtem oder erniedrigtem Druck zu arbeiten.

Als Wittig-Reagenzien eignen sich die üblichen Reagenzien. Bevorzugt ist 3-Trifluormethylbenzyltriphenylphosphoniumbromid.

Als Basen eignen sich im allgemeinen eine der oben aufgeführten Basen, vorzugsweise Li-bis-(triethylbutyl)amid.

Die Base wird in einer Menge von 0,1 mol bis 5 mol, bevorzugt von 0,5 mol bis 2 mol jeweils bezogen auf 1 mol der Ausgangsverbindung eingesetzt.

Die Umsetzung mit Wittig-Reagenzien wird im allgemeinen in einem Temperaturbereich von 0°C bis 150°C, bevorzugt bei 25°C bis 40°C, durchgeführt.

Die Wittig-Reaktionen werden im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Die Verbindungen der allgemeinen Formel (II) sind bekannt oder können hergestellt werden, indem man Verbindungen der allgemeinen Formel (IV) 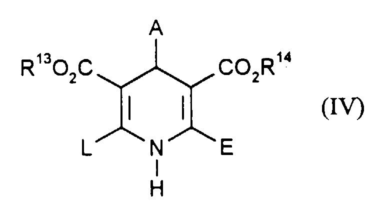 in welcher
- A, E und L: die oben angegebene Bedeutung haben,
- R¹³ und R¹⁴: gleich oder verschieden sind und für geradkettiges oder verzweigtes Alkyl mit bis zu 4 Kohlenstoffatomen stehen,
mit Oxidationsmitteln in inerten Lösemitteln oxidiert
und
in einem zweiten Schritt selektiv die Alkoxycarbonylfunktion (CO₂R¹³) zur Hydroxyfunktion reduziert.

Als Lösemittel eignen sich für die Oxidation Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Dichlormethan.

Als Oxidationsmittel eignen sich beispielsweise 2,3-Dichlor-5,6-dicyan-benzochinon, Pyridiniumchlorochromat (PCC), Osmiumtetroxid und Mangandioxid. Bevorzugt ist für den oben aufgeführten Schritt 2,3-Dichlor-5,6-dicyan-benzochinon (DDQ).

Das Oxidationsmittel wird in einer Menge von 1 mol bis 10 mol, bevorzugt von 2 mol bis 5 mol bezogen auf 1 mol der Verbindungen der allgemeinen Formel (IV) eingesetzt.

Die Oxidation verläuft im allgemeinen in einem Temperaturbereich von -50°C bis +100°C, bevorzugt von 0°C bis Raumtemperatur.

Die Oxidation verläuft im allgemeinen bei Normaldruck. Es ist aber auch möglich, die Oxidation bei erhöhtem oder erniedrigtem Druck durchzuführen.

Die 1,4-Dihydropyridin-3,5-dicarbonsäureester der allgemeinen Formel (IV) sind bekannt oder können nach bekannten Methoden hergestellt werden.

Die Verbindungen der allgemeinen Formel (III) sind größtenteils neu und werden hergestellt, indem man
Verbindungen der allgemeinen Formel (V) 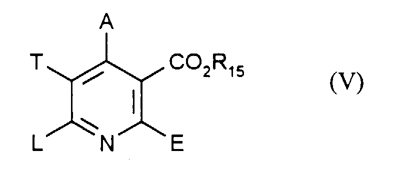 in welcher
- A, E, L und T: die oben angegebene Bedeutung haben
und
- R¹⁵: für geradkettiges oder verzweigtes Alkoxycarbonyl mit bis zu 3 Kohlenstoffatomen steht,
zunächst durch Reduktion der Alkoxycarbonylfunktion in die Verbindungen der allgemeinen Formel (Ia) 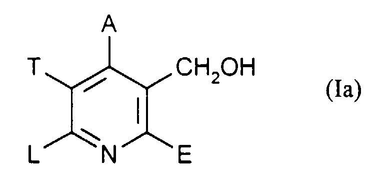 in welcher
- A, E, L und T: die oben angegebene Bedeutung haben,
überführt,
und in einem zweiten Schritt die Hydroxymethylfunktion nach den oben aufgeführten Bedingungen, vorzugsweise mit Pyridiniumchlorochromat (PCC) zum Aldehyd oxidiert.

Die einzelnen Reaktionsschritte werden im allgemeinen in einem Temperaturbereich von -10°C bis +160°C, vorzugsweise von 0°C bis +100°C und Normaldruck durchgeführt.

Die Verbindungen der allgemeinen Formel (V) werden in Analogie zu der oben beschriebenen Methode zur Herstellung der Verbindungen der allgemeinen Formel (II) hergestellt.

Die Verbindungen der allgemeinen Formel (Ia) sind neu und können wie oben beschrieben hergestellt werden.

Die erfindungsgemäßen Verbindungen der allgemeinen Formeln (I) und (Ia) haben ein nicht vorhersehbares pharmakologisches Wirkspektrum.

Die erfindungsgemäßen Verbindungen der allgemeinen Formeln (I) und (Ia) besitzen wertvolle, im Vergleich zum Stand der Technik überlegene, pharmakologische Eigenschaften, insbesondere sind sie hochwirksame Inhibitoren des Cholesterin-Ester-Transfer-Proteins (CETP) und stimulieren den Reversen Cholesterintransport. Die erfindungsgemäßen Wirkstoffe bewirken eine Senkung des LDL-Cholesterinspiegels im Blut bei gleichzeitiger Erhöhung des HDL-Cholesterinspiegels. Sie können deshalb zur Behandlung von Hyperlipoproteinämie, Dislipidämie, Hypertriglyceridämie, kombinierte Hyperlipidämie oder Arteriosklerose eingesetzt werden.

Die Erfindung betrifft außerdem die Kombination von erfindungsgemäßen 2-Arylsubstituierten Pyridinen der allgemeinen Formel (I) mit einem Glucosidase- und/oder Amylasehemmer zur Behandlung von familiärer Hyperlipidaemien, der Fettsucht (Adipositas) und des Diabetes mellitus. Glucosidase- und/oder Amylasehemmer im Rahmen der Erfindung sind beispielsweise Acarbose, Adiposine, Voglibose, Miglitol, Emiglitate, MDL-25637, Camiglibose (MDL-73945), Tendamistate, AI-3688, Trestatin, Pradimicin-Q und Salbostatin.

Bevorzugt ist die Kombination von Acarbose, Miglitol, Emiglitate oder Voglibose mit einer der oben aufgeführten erfindungsgemäßen Verbindungen der allgemeinen Formel (I).

Weiterhin können die erfindungsgemäßen Verbindungen in Kombination mit Cholesterin senkenden Vastatinen oder Apo B senkenden Prinzipien kombiniert werden, um Dyslipidämien, kombinierte Hyperlipidämien, Hypercholesterolämien oder Hypertriglyceridämien zu behandeln.

Die genannten Kombinationen sind auch zur primären oder sekundären Prävention koronarer Herzerkrankungen (z.B. Myokardinfarkt) einsetzbar.

Vastatine im Rahmen der Erfindung sind beispielsweise Lovastatin, Simvastatin, Pranastatin, Fluvastatin, Atorvastatin und Cerivastatin. Apo B senkende Mittel sind zum Beispiel MTP-Inhibitoren.

Bevorzugt ist die Kombination von Cerwastatin oder Apo B-Inhibitoren mit einer der oben aufgeführten erfindungsgemäßen Verbindungen der allgemeinen Formel (I).

Die pharmakologische Wirkung der erfindungsgemäßen Stoffe wurde in folgendem Test bestimmt:

### CETP-Inhibitions-Testung

### 1. Gewinnung von CETP

CETP wird aus humanem Plasma durch Differential-Zentrifugation und Säulenchromatographie in partiell gereinigter Form gewonnen und zum Test verwendet. Dazu wird humanes Plasma mit NaBr auf eine Dichte von 1,21 g pro ml eingestellt und 18 h bei 50.000 Upm bei 4°C zentrifugiert. Die Bodenfraktion (d>1,21 g/ml) wird auf eine Sephadex®Phenyl-Sepharose 4B (Fa. Pharmacia) Säule aufgetragen, mit 0,15 m NaCl/0,001 m TrisHCl pH 7,4 gewaschen und anschließend mit dest. Wasser eluiert. Die CETP-aktiven Fraktionen werden gepoolt, gegen 50mM Na Acetat pH 4,5 dialysiert und auf eine CM-Sepharose® (Fa. Pharmacia)-Säule aufgetragen. Mit einem linearen Gradienten (0-1 M NaCl) wird anschließend eluiert. Die gepoolten CETP-Fraktionen werden gegen 10 mM TrisHCl pH 7,4 dialysiert und anschließend durch Chromatographie über eine Mono Q®-Säule (Fa. Pharmacia) weiter gereinigt.

### 2. Gewinnung von radioaktiv markiertem HDL

50 ml frisches humanes EDTA-Plasma wird mit NaBr auf eine Dichte von 1,12 eingestellt und bei 4°C im Ty 65-Rotor 18 h bei 50.000 Upm zentrifugiert. Die Oberphase wird zur Gewinnung von kaltem LDL verwendet. Die Unterphase wird gegen 3*4 l PDB-Puffer (10 mM Tris/HCl pH 7,4, 0,15 mM NaCl, 1 mM EDTA, 0,02% NaN₃) dialysiert. Pro 10 ml Retentatvolumen wird anschließend 20 µl ³H-Cholesterin (Dupont NET-725; 1 µC/µl gelöst in Ethanol) hinzugesetzt und 72 h bei 37°C unter N₂ inkubiert.

Der Ansatz wird dann mit NaBr auf die Dichte 1,21 eingestellt und im Ty 65-Rotor 18 h bei 50.000 Upm bei 20°C zentrifugiert. Man gewinnt die Oberphase und reinigt die Lipoproteinfraktionen durch Gradientenzentrifugation. Dazu wird die isolierte, markierte Lipoproteinfraktion mit NaBr auf eine Dichte von 1,26 eingestellt. Je 4 ml dieser Lösung werden in Zentrifugenröhrchen (SW 40-Rotor) mit 4 ml einer Lösung der Dichte 1,21 sowie 4,5 ml einer Lösung von 1,063 überschichtet (Dichtelösungen aus PDB-Puffer und NaBr) und anschließend 24 h bei 38.000 Upm und 20°C im SW 40-Rotor zentrifugiert. Die zwischen der Dichte 1,063 und 1,21 liegende, das markierte HDL enthaltende Zwischenschicht wird gegen 3*100 Volumen PDB-Puffer bei 4°C dialysiert.
Das Retentat enthält radioaktiv markiertes ³H-CE-HDL, das auf ca. 5x10⁶ cpm pro ml eingestellt zum Test verwendet wird.

### 3. Testdurchführung

Zur Testung der CETPAktivität wird die Übertragung von ³H-Cholesterol-ester von humanen HD-Lipoproteinen auf biotinylierte LD-Lipoproteine gemessen.
Die Reaktion wird durch Zugabe von Streptavidin-SPA®beads (Fa. Amersham) beendet und die übertragene Radioaktivität direkt im Liquid Scintillation Counter bestimmt.
Im Testansatz werden 10 µl HDL-³H-Cholesterolester (∼ 50.000 cpm) mit 10 µl Biotin-LDL (Fa. Amersham) in 50 mM Hepes / 0,15 m NaCl / 0,1% Rinderserumalbumin / 0,05% NaN₃ pH 7,4 mit 10 µl CETP (1 mg/ml) und 3 µL Lösung der zu prüfenden Substanz (in 10% DMSO/ 1% RSA gelöst), für 18 h bei 37°C inkubiert. Anschließend werden 200 µl der SPA-Streptavidin-Bead-Lösung(Amersham TRKQ 7005) zugesetzt, 1 h unter Schütteln weiter inkubiert und anschließend im Scintillationszähler gemessen. Als Kontrollen dienen entsprechende Inkubationen mit 10 µl Puffer, 10 µl CETP bei 4°C sowie 10 µl CETP bei 37°C.
Die in den Kontrollansätzen mit CETP bei 37°C übertragene Aktivität wird als 100% Übertragung gewertet. Die Substanzkonzentration, bei der diese Übertragung auf die Hälfte reduziert ist, wird als IC₅₀-Wert angegeben.

In der folgenden Tabelle sind die IC₅₀-Werte (mol/l) für CETP-Inhibitoren angegeben:

| **Beispiel-Nr.** | **IC**_{**50**}**-Wert (mol/l)** |
|---|---|
| 3 | 8 x 10⁻⁷ |
| 14 | 7 x 10⁻⁸ |
| 26 | 9 x 10⁻⁷ |
| 31 | 5 x 10⁻⁷ |
| 37 | 3 x 10⁻⁷ |
| 42 | 1,7 x 10⁻⁷ bis 9 x 10⁻⁸ |
| 45 | 3 x 10⁻⁷ |
| 51 | 4 x 10⁻⁷ |
| 53 | 1,7 x 10⁻⁷ |
| 63 | 1,0 x 10⁻⁶ |
| 68 | 8 x 10⁻⁸ |
| 83 | 5 x 10⁻⁶ |

Syrische Goldhamster aus werkseigener Zucht werden nach 24-stündigem Fasten narkotisiert (0,80 mg/kg Atropin, 0,80 mg/kg Ketavet® s.c., 30' später 50 mg/kg Nembutal i.p.). Anschließend wird die V.jugularis freipräpariert und kanüliert. Die Testsubstanz wird in einem geeigneten Lösemittel (in der Regel Adalat-Placebolösung: 60 g Glycerin, 100 ml H₂O, ad 1000 ml PEG-400) gelöst und den Tieren über einen in die V.jugularis eingeführten PE-Katheter verabreicht. Die Kontrolltiere erhalten das gleiche Volumen Lösungsmittel ohne Testsubstanz. Anschließend wird die Vene abgebunden und die Wunde verschlossen. Nach verschiedenen Zeitpunkten - bis zu 24 Stunden nach Applikation der Testsubstanz - wird den Tieren durch Punktion des retro-orbitalen Venenplexus Blut entnommen (ca. 250 µl). Durch Inkubation bei 4°C über Nacht wird die Gerinnung abgeschlossen, anschließend wird 10 Minuten bei 6000 g zentrifugiert. Im so erhaltenen Serum wird der Gehalt an Cholesterin und Triglyceriden mit Hilfe modifizierter kommerziell erhältlicher Enzymtests bestimmt (Cholesterin enzymatisch 14366 Merck, Triglyceride 14364 Merck). Serum wird in geeigneter Weise mit physiologischer Kochsalzlösung verdünnt.

100 µl Serum-Verdünnung werden mit 100 µl Testsubstanz in 96-Lochplatten versetzt und 10 Minuten bei Raumtemperatur inkubiert. Anschließend wird die optische Dichte bei einer Wellenlänge von 492 nm mit einem automatischen Platten-Lesegerät bestimmt (SLT-Spectra). Die in den Proben enthaltene Triglycerid/Cholesterinkonzentration wird mit Hilfe einer parallel gemessenen Standardkurve bestimmt.

Die Bestimmung des Gehaltes von HDL-Cholesterin wird nach Präzipitation der ApoB-haltigen Lipoproteine mittels eines Reagenziengemisch (Sigma 352-4 HDL Cholesterol Reagenz) nach Herstellerangaben durchgeführt.

Bei Versuchen zur Bestimmung der oralen Wirksamkeit wird syrischen Goldhamstern aus werkseigener Zucht Testsubstanz in DMSO gelöst und 0,5% Tylose suspendiert mittels Schlundsonde peroral verabreicht. Die Kontrolltiere erhalten identische Volumen Lösemittel ohne Testsubstanz. Anschließend wird den Tieren das Futter entzogen und zu verschiedenen Zeitpunkten - bis zu 24 Stunden nach Substanzapplikation - durch Punktion des retroorbitalen Venenplexus Blut entnommen. Die weitere Bearbeitung erfolgt wie oben beschrieben.

### Bestimmung der CETP-Aktivität

Nach intravenöser Applikation von 20 mg/kg der Verbindung aus Beispiel 42 findet sich eine ca. 50%ige Reduktion der ex vivo gemessenen CETP-Aktitivät über einen Zeitraum von mindestens 2 Stunden. 24 Stunden nach Substanzapplikation sind die HDL-Cholesterinwerte in der substanzbehandelten Gruppe ca. 30% höher als in der Kontrollgruppe. Ebenso finden sich 24 Stunden nach Applikation von 100 mg/kg per os der Verbindung aus Beispiel 42 um 30% erhöhte HDL-Cholesterinspiegel in der substanzbehandelten Gruppe.

Nach Applikation von 2 x 45 mg/kg per os der Verbindung aus Beispiel 44 ist der Spiegel von HDL-Cholesterin 24 Stunden nach der ersten Applikation um 20% gegenüber der Kontrollgruppe erhöht. Zu diesem Zeitpunkt ist der Spiegel der Triglyceride um 70% gegenüber der Kontrollgruppe erniedrigt.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise oral oder parenteral, insbesondere perlingual oder intravenös, vorzugsweise oral.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehreren Einzelgaben über den Tag zu verteilen.

### I. Laufmittel für DC

- A₁ =: Petrolether 40/60 : Essigester 4:1
- A₂ =: Petrolether 40/60 : Essigester 6:1
- A₃ =: Petrolether 40/60 : Essigester 9:1
- A₄ =: Toluol
- A₅ =: Toluol : Essigester 9:1
- A₆ =: Petrolether 40/60 : Essigester 2:1
- A₇ =: Petrolether : Essigester 5:1
- A₈ =: Toluol : Essigester 7:3
- A₉ =: Cyclohexan / Tetrahydrofuran 8:2
- A₁₀ =: Cyclohexan / Tetrahydrofuran 9:1
- A₁₁ =: Toluol : Essigester 8:2

### Ausgangsverbindungen

### Beispiel I

### 1,4-Dihydro-2,4-bis-(4-fluorphenyl)-6-isopropyl-pyridin-3,5-dicarbonsäure-3-methyl-5-ethylester

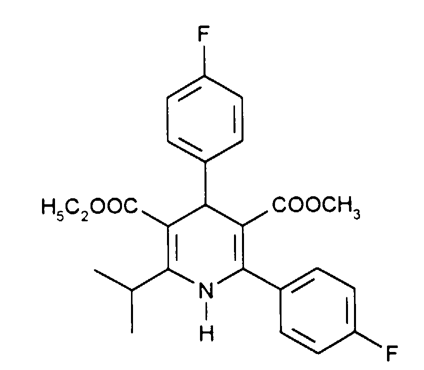

31 g (0,25 mol) p-Fluorbenzaldehyd, 49 g (0,25 mol) 4-Fluorbenzoylessigsäure-methylester und 39,25 g (0,25 mol) 3-Amino-4-methyl-pent-2-en-säure-ethylester werden in 150 ml Ethylenglykol bei einer Badtemperatur von 130°C 18 Stunden unter Rückfluß gekocht. Die Mischung wird auf Raumtemperatur abgekühlt, dreimal mit 300 ml Diethylether extrahiert, die vereinigten Etherphasen eingeengt, der Rückstand in 100 ml Toluol gelöst und an 700 ml Kieselgel (0,04 - 0,063 mm) mit Toluol als Elutionsmittel chromatographiert.
Ausbeute: 21,52 g (19,5% d.Th.)
R_{f} = 0,29 (A₅)

### Beispiel II

### 2,4-Bis-(4-fluorphenyl)-6-isopropyl-4-pyridin-3,5-dicarbonsäure-3-methyl-5-ethylester

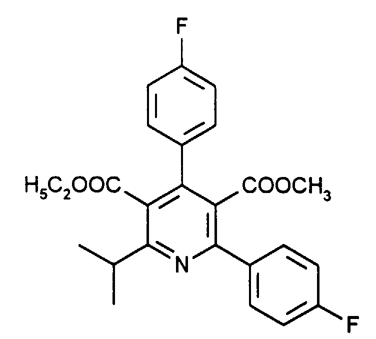

Zu einer Lösung von 6,0 g (13,59 mmol) der Verbindung aus Beispiel I in 180 ml CH₂Cl₂ p.a. gibt man 3,08 g (13,59 mmol) 2,3-Dichlor-5,6-dicyan-p-benzochinon (DDQ) und rührt 1 h bei Raumtemperatur. Dann wird über Kieselgel 60 abgesaugt, mit 200 ml CH₂Cl₂ nachgewaschen, die vereinigten Filtrate bis zu einem Öl eingeengt, das in Petrolether kristallisiert.
Ausbeute: 3,96 g (66,3% d.Th.)
R_{f} = 0,54 (A₅)

### Beispiel III

### 2,4-Bis-(4-fluorphenyl)-6-isopropyl-5-hydroxymethyl-pyridin-3-carbonsäuremethylester

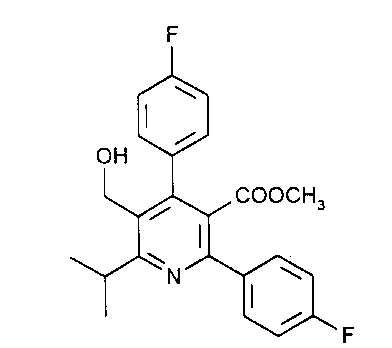

Unter Argon gibt man zu einer Lösung von 34, 5 g (0,0811 mol) der Verbindung aus Beispiel II in 300 ml THF p.a. 47,5 ml (0,166 mol, 2,05 eq.) einer 3,5-molaren Lösung von Natrium-bis-(2-methoxyethoxy)-dihydroaluminat (Red-Al) in Toluol bei Raumtemperatur langsam hinzu und rührt 3 h nach. Die Reaktionslösung wird unter Eiskühlung mit 150 ml einer 20%igen Kaliumnatriumtartrat-Lösung versetzt und mit 200 ml Essigester extrahiert. Die organische Phase wird einmal mit gesättigter NaCl-Lösung gewaschen, über Na₂SO₄ getrocknet und im Vakuum eingeengt. Der Rücktand wird an Kieselgel 60 zunächst mit Toluol, danach mit einem steigenden Anteil an Essigester (Toluol / Essigester = 9/1) chromatographiert. Die Eluate werden eingeengt und durch Verreiben mit Petrolether kristallisiert.
Ausbeute: 12,8 g (39,8% d.Th.)
R_{f} = 0,29 (A₅)

### Beispiel IV

### 2,4-Bis-(4-fluorphenyl)-6-isopropyl-5-formyl-pyridin-3-carbonsäure-methylester

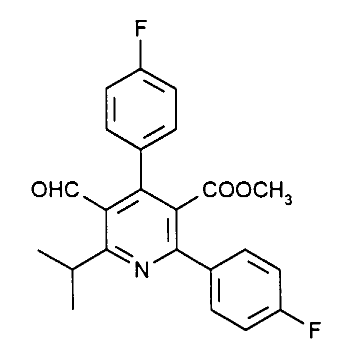

Zu einer Lösung von 30,3 g (0,0762 mol) der Verbindung aus Beispiel III in 500 ml CH₂Cl₂ gibt man 15,55 g (0,1525 mol; 2,0 eq.) neutrales Al₂O₃ und 32,93 g (0,1525 mol; 2 eq.) Pyridiniumchlorochromat (PCC) und rührt 1h bei Raumtemperatur. Man saugt über Kieselgel ab, wäscht mit 600 ml CH₂Cl₂ nach und engt das Filtrat im Vakuum ein, wobei das Produkt auskristallisiert.
Ausbeute: 13,9 g (90,1% d.Th.)
R_{f} = 0,8 (A₁₁)

### Beispiel V

### 4,6-Bis-p-fluorphenyl-2-isopropyl-3-[(p-trifluormethylphenyl)-hydroxymethyl]-pyridin-5-carbonsäure-methylester

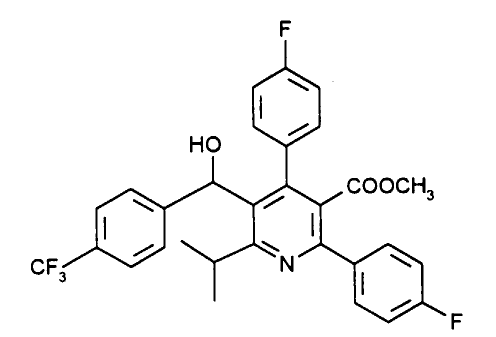

215,8 mg (8,88 mmol) Magnesiumspäne werden in 30 ml THF p.a. vorgelegt, unter Argon zum Rückflußkochen erhitzt und 1,34 ml (9,56 mmol; 1,4 eq.) 4-Brombenzotrifluorid mittels Spritze rasch zugetropft. Nach anfänglich sehr heftiger Reaktion wird 30 min unter Rückfluß gekocht; danach läßt man auf Raumtemperatur abkühlen (Grignard-Reagenz). 2,7 g (6,83 mmol) der Verbindung aus Beispiel IV werden in 20 ml THF p.a. gelöst, unter Argon auf -78°C gekühlt und dann das Grignard-Reagenz mittels Spritze addiert. Danach wird der Ansatz ohne Kühlung 45 min gerührt. Die Reaktionslösung wird in 250 ml Essigester / NH₄Cl-Lösung (1:1) verteilt, die organische Schicht abgetrennt, die wäßrige Schicht zweimal mit Essigester extrahiert. Die vereinigten organischen Phasen werden über Na₂SO₄ getrocknet und im Vakuum eingeengt. Der ölige Rückstand wird mit Petrolether kristallisiert, abgesaugt und im Vakuum getrocknet.
Ausbeute: 2,55 g (69% d.Th.)
R_{f} = 0,42 (A₅)

### Beispiel VI

### 2,4-Bis-(4-fluorphenyl)-6-isopropyl-5-[4-(trifluorphenyl)-fluormethyl]-pyridin-3-carbonsäure-methylester

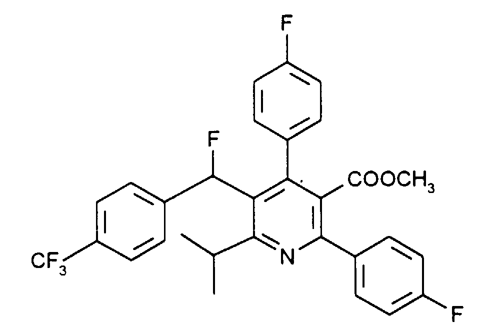

Zu einer Lösung von 600 mg (1,108 mmol) der Verbindung aus Beispiel V in 15 ml CH₂Cl₂ p.a. werden bei -70°C unter Argon 0,22 ml (1,66 mmol, 1,5 eq.) Diethylaminoschwefeltrifluorid (DAST) mittels Spritze zugegeben, das Kältebad entfernt und 1 h bei -5°C gerührt. Danach wird die Reaktionslösung in Essigester / NH₄Cl-Lösung verteilt, die organische Phase abgetrennt und die wäßrige Phase einmal mit Essigester gewaschen. Die vereinigten Essigesterphasen werden über Na₂SO₄ getrocknet, im Vakuum eingeengt und der Rückstand durch Verreiben mit Petrolether kristallisiert.
Ausbeute: 359 mg (59,6% d.Th.)
R_{f} = 0,79 (A₅)

### Beispiel VII

### 2,4-Bis-(4-fluorphenyl)-6-isopropyl-5-[(4-(trifluormethylphenyl)chlor-methyl]-pyridin-3-carbonsäure-methylester

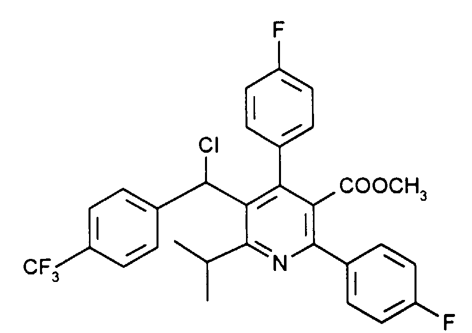

1,85 g (3,416 mmol) der Verbindung aus Beispiel V werden in 500 ml CH₂Cl₂ p.a. unter Argon gelöst, auf -40°C gekühlt und 0,745 ml (10,25 mmol; 3,0 eq.) SOCl₂ mittels Spritze zugetropft. Man rührt noch 30 min bei -40°C und lagert anschließend den Ansatz bei -30°C über Nacht. Danach wird bei Raumtemperatur (35 min.) weitergerührt bis zum vollständigen Umsatz (DC). Es wird auf 100 ml gesättigte NaHCO₃-Lösung und 200 ml Essigester gegossen, die organische Phase abgetrennt, einmal mit 50 ml gesättigter NaHCO₃-Lösung gewaschen, über Na₂SO₄ getrocknet, im Vakuum eingeengt und der Rückstand durch Verreiben mit Petrolether kristallisiert.
Ausbeute: 1,42 g (74,6% d.Th.)
R_{f} = 0,9 (A₅)

### Beispiel VIII

### 2-Bis-(4-fluorphenyl)-6-isopropyl-5-[4-(trifluormethylphenyl)-azidomethyl]pyridin-3-carbonsäure-methylester

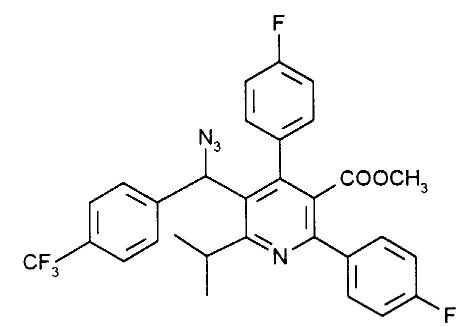

0,9 g (1,607 mmol) der Verbindung aus Beispiel VII und 1,044 g (16,07 mmol; 10 eq.) NaN₃ werden in 40 ml DMSO 4 h bei 80°C und danach 12 h bei Raumtemperatur gerührt. Anschließend wird mit 100 ml Essigester versetzt, einmal mit Wasser und dreimal mit gesättigter NaCl-Lösung gewaschen. Die organische Phase wird über Na₂SO₄ getrocknet, eingeengt und der Rückstand durch Verreiben mit Petrolether kristallisiert.
Ausbeute: 370 mg (40,7% d.Th.)
R_{f} = 0.81 (A₅)

### Beispiel IX

### 2,4-Bis-(4-fluorphenyl)-6-isopropyl-5-[4-(trifluormethylphenyl)-aminomethyl]-pyridin-3-carbonsäure-methylester

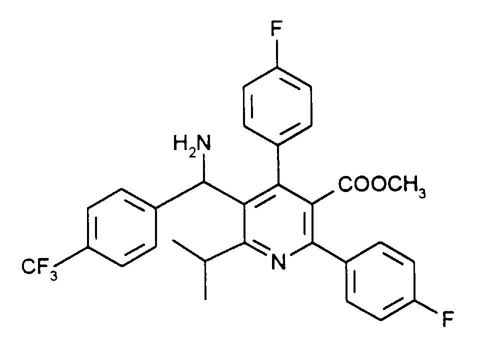

630 mg (1,112 mmol) der Verbindung aus Beispiel VIII werden in 40 ml abs. CH₃OH und 20 ml abs. THF in Gegenwart von 60 mg Pd/C (10%) mit Wasserstoff bei Normaldruck und 20°C über Nacht hydriert. Danach wird der Katalysator über Kieselgel abgesaugt, mit Methanol / THF (1:1) und THF nachgewaschen, und das Lösemittel im Vakuum abdestilliert. Der Rückstand wird über 70 g Kieselgel durch Säulenchromatographie mit Toluol und Toluol / Essigester (7:3) gereinigt.
Ausbeuten:
1. Fraktion = 113 mg (18,8% d.Th.)
2. Fraktion = 296 mg (49,3% d.Th.)
R_{f} = 0,13 (A₅)

### Beispiel X

### 2,4-Bis-(4-fluorphenyl)-6-cyclopentyl-5-[2-(benzothiazol-2-yl)-hydroxymethyl]-pyridin-3-carbonsäure-methylester

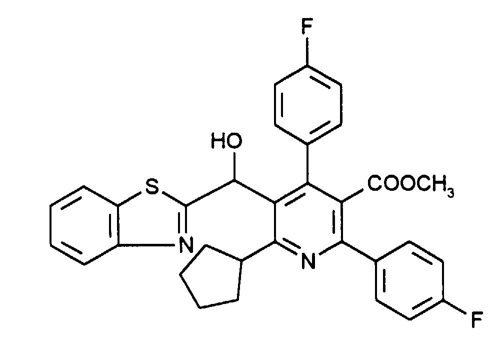

Zu 230 mg (1,7 mmol) Benzothiazol in 20 ml THF p.a. gibt man unter Argon bei -78°C 1.04 ml (1,7 mmol) Butyllithium (1,6 molar in Hexan). Nach 5 min Rühren bei -78°C tropft man eine Lösung von 623 mg (1,478 mol) 2,4-Bis-(4-fluorphenyl)-6-cyclopentyl-5-formyl-pyridin-3-carbonsäure-methylester in 10 ml THF p.a. hinzu. Danach läßt man von -78°C bis auf Raumtemperatur ansteigen. Es wird auf Essigester / NH₄Cl-Lösung unter Eiskühlung gegossen, die organische Phase einmal mit gesättigter NaCl-Lösung gewaschen, über Na₂SO₄ getrocknet und eingeengt. Nach Chromatographie an 50 ml Kieselgel mit Toluol erhält man 466 mg (56,7% d.Th.).
R_{f} = 0,33 (A₅)

### Beispiel XI

### 2,4-Bis-(4-fluorphenyl)-6-cyclopentyl-5-[2-(benzothiazol-2-yl)-fluormethyl]-pyridin-3-carbonsäure-methylester

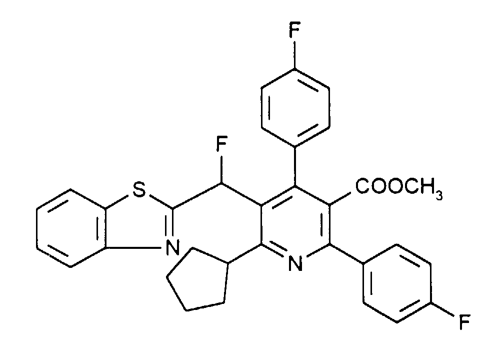

Analog Beispiel VI werden 439 mg (0,789 mmol) der Verbindung aus Beispiel X mit 190,7 mg (1,183 mmol; 1,5 eq.) DAST in 30 ml CH₂Cl₂ p.a. umgesetzt.
Ausbeute: 350 mg (79,5% d.Th.)
R_{f} = 0,47 (A₅)

### Beispiel XII

### 2,4-Bis-(4-fluorphenyl)-6-isopropyl-5-[2-(E/Z)-3-(trifluor-methylphenyl)-vinyl-pyridin-3-carbonsäure-methylester

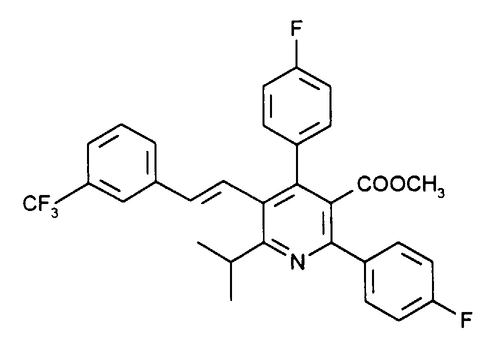

1,755 g (3,5 mmol) 3-Trifluormethylbenzyl-triphenylphosphoniumbromid werden in 10 ml THF p.a. unter Argon bei 0°C tropfenweise mit 3,85 ml (3,85 mmol; 1,1 eq.) Lithium-bis-(trimethylsilyl)amid (1,0 molar in Hexan) innerhalb von 10 min versetzt und 30 min bei 0°C gerührt. Dann gibt man 1,246 mg (3,15 mmol; 0,9 eq.) der Verbindung aus Beispiel IV in 2 ml THF tropfenweise in 10 min bei 0°C hinzu und rührt 20 min bei 0°C und 80 min ohne Kühlung. Die Reaktionslösung wird mit gesättigter NH₄Cl-Lösung versetzt und dreimal mit je 40 ml Essigester extrahiert. Die vereinigte Essigesterphasen werden über Na₂SO₄ getrocknet und im Vakuum eingeengt. Der Rückstand wird an 60 ml Kieselgel 60 mit Toluol chromatographiert.
Ausbeute 1,22 g (72,1% d.Th.)
R_{f} = 0,77 (A₄)

### Beispiel XIII

### 2,4-Bis-(4-fluorphenyl)-6-isopropyl-5-[2-(3-trifluormethyl-phenyl)-1,2-di-hydroxyethyl]-pyridin-3-carbonsäure-methylester

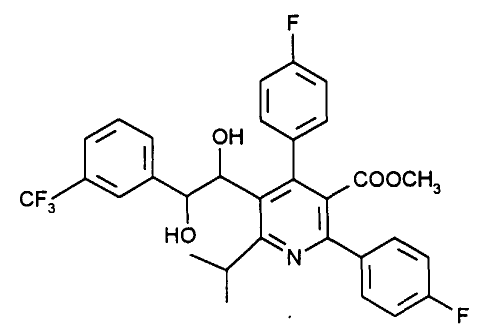

Eine Mischung von 300mg (0,559 mmol) der Verbindung aus Beispiel XII, 141 mg (1,2 mol; 2,1 eq) N-Methylmorpholin-N-oxid und 2,1 ml (0,168 mmol) einer 2,5%igen Osmiumtetroxid-Lösung in tert.Butanol (≙ 0,08 mol OsO₄ x 1⁻¹) wird bei Raumtemperatur über Nacht im Dunkeln (Ansatz mittels Alu-Folie umwickelt) gerührt. Nach Zugabe von 130 mg (1 mmol) Na₂SO₃ wird die Reaktionslösung mit 30 ml CH₂Cl₂, 10 ml NaCl-Lösung und 10 ml Wasser verdünnt. Die CH₂Cl₂-Phase wird abgetrennt, einmal mit NaCl-Lösung und Wasser gewaschen, über Na₂SO₄ getrocknet, filtriert und eingeengt. Der ölige Rückstand wird durch Chromatographie an 50 ml Kieselgel 60 mit Toluol und Toluol/Essigester (8:2) gereinigt.
Ausbeute: 140 mg (43,8% d.Th.)
R_{f} = 0,18 (A₅)

### Beispiel XIV

### 4,6-Bis-p-fluorphenyl-2-isopropyl-3-(p-trifluormethyl)-benzoyl-5-pyridin-carbaldehyd

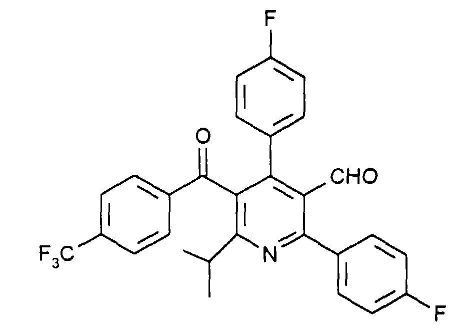

Eine Lösung von 1,3 g (2,5 mmol) 4,6-Bis-p-fluorphenyl-5-hydroxymethyl-2-isopropyl-3-[(p-trifluormethylphenyl)-(hydroxy)-methyl]-pyridin in 50 ml CH₂Cl₂ wird bei RT mit einer Mischung aus 1 g (10 mmol) Al₂O₃ und 2,2 g (10 mmol) PCC versetzt und über Nacht bei RT gerührt. Zur Aufarbeitung wird Kieselgel zugegeben, 20 min bei RT gerührt, über Kieselgel filtriert, mit CH₂Cl₂ gewaschen und das Filtrat eingeengt.
Ausbeute: 1,04 g (82% d.Th.)
R_{f} = 0,46 (Toluol)

### Beispiel XV

### 4,6-Bis-p-fluorphenyl-2-isopropyl-3-[{1-(4-fluornaphthyl)}-(methoxy)methyl]-5-pyridincarbonsäuremethylester

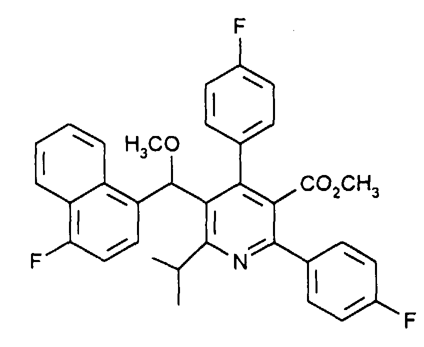

Zu einer Suspension von 55 mg NaH (80%) in 20 ml DMF wird unter Argon bei -10°C eine Lösung von 1 g (1,8 mmol) 4,6-Bis-p-fluorphenyl-2-isopropyl-3-[{1-(4-fluornaphthyl}-(hydroxy)methyl]5-pyridin-carbonsäuremethylester in 10 ml DMF zugetropft und 20 min gerührt. Anschließend wird bei -10°C eine Lösung von 0,14 ml (2,3 mmol) CH₃I in 5 ml DMF zugetropft. Nach 1 h Rühren wird langsam aufgetaut und 2 h bei RT nachgerührt. Zur Aufarbeitung wird mit 20 ml 1 N AcOH versetzt, dreimal mit Essigester extrahiert, und die vereinigten organischen Phasen werden über Na₂SO₄ getrocknet, filtriert und eingeengt. Das Produkt wird über Kieselgel 60 chromatographiert.
Ausbeute: 0,95 g (95% d.Th.)
R_{f} = 0,53 (Toluol)

### Beispiel XVI

### 4,6-Bis-p-fluorphenyl-2-isopropyl-3-[(p-trifluormethylphenyl)(fluoro)methyl]5-pyridincarbaldehyd

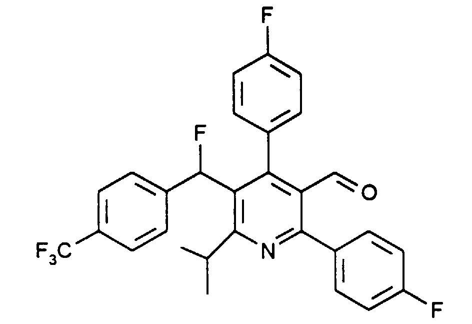

9,0 g (17,5 mmol) der Verbindung 2,4-Bis-(4-fluorphenyl)-6-isopropyl-5-[4-trifluormethylphenyl)-fluormethyl]-3-hydroxymethyl-pyridin (Beispiel 31) werden in 200 ml CH₂Cl₂ bei RT mit 3,56 g (34,9 mmol) Al₂O₃ und 7,68 g (34,9 mmol) PCC 2 h gerührt. Anschließend wird Kieselgel zugegeben, 10 min gerührt, über Kieselgel filtriert, mit CH₂Cl₂ nachgewaschen und eingeengt.
Ausbeute: 7,49 g (84% d.Th.)
R_{f} = 0,76 (Toluol)

### Beispiel XVII

### 4,6-Bis-p-fluorphenyl-2-isopropyl-3-[(p-trifluormethylphenyl)-(fluoro)-methyl]-pyridin-5-ω-propensäuremethylester

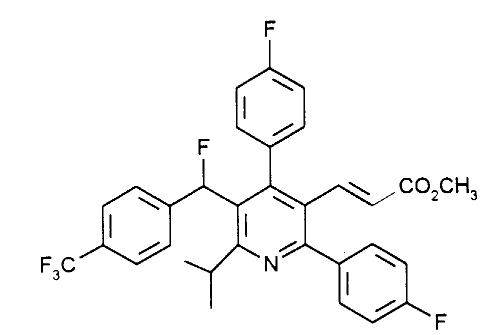

0,19 ml Diisopropylamin werden bei -78°C in 10 ml THF unter Argon mit 0,78 ml 1,6 N (1,25 mmol) n-BuLi versetzt, 5 min bei 0°C gerührt, bei -78°C mit 0,09 ml (1,1 mmol) Methylacetat versetzt und 30 min gerührt. Anschließend werden 0,40 g (0,78 mmol) der Verbindung aus Beispiel XVI in 10 ml THF gelöst zugetropft. Nach 4 h Rühren bei -78°C wird über Nacht aufgetaut. Unter Kühlung wird mit NH₄Cl-Lösung und Wasser versetzt und zweimal mit Essigester extrahiert. Die vereinigten organischen Phasen werden über Natriumsulfat getrocknet, eingeengt und über Kieselgel 60 (Petrolether / Essigester = 10/1) chromatographiert.
Ausbeute: 0,11 g (25% d.Th.)
R_{f} = 0,35 (Petrolether / Essigester = 9/1)

### Beispiel XVIII

### 4,6-Bis-p-fluorphenyl-2-isopropyl-3-p-trifluormethylphenyl-benzoyl-5-pyridinacetaldehyd-methylenolether

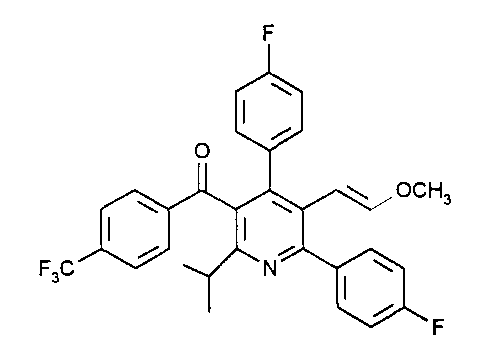

5,7 g (13,2 mmol) Methoxymethyl-triphenylphosphonium-bromid / Natriumamid (Instant-Ylid) werden in 100 ml THF suspendiert, bei RT 20 min gerührt, tropfenweise mit einer Lösung von 2,7 g (5,3 mmol) der Verbindung aus Beispiel XIV in 50 ml THF versetzt und über Nacht gerührt. Zur Aufarbeitung wird auf Eiswasser gegeben, dreimal mit CH₂Cl₂ extrahiert, und die vereinigten organischen Phasen werden getrocknet, filtriert, eingeengt und über Kieselgel 60 (Petrolether / Essigester = 9/1) chromatographiert.
Ausbeute: 1,0 g (35% d.Th.)
R_{f} = 0,6 (Petrolether / Essigester = 9/1)

### Beispiel XIX

### 4,6-Bis-p-fluorphenyl-2-isopropyl-3-p-trifluormethyl-benzoyl-5-pyridin-acetaldehyd

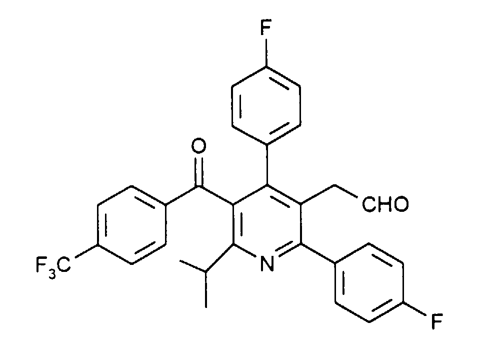

Zu einer Lösung von 0,7 g (1,3 mmol) der Verbindung aus Beispiel XVIII in 30 ml CH₃CN werden unter Argon 0,2 g (1,3 mmol) NaI und 0,14 g (1,3 ml) (CH₂)₃SiCl zugegeben und 3 h bei RT gerührt. Nach erneuer Zugabe der gleichen Mengen NaI und Me₃SiCl wird über Nacht gerührt. Anschließend wird mit gesättigter wäßriger Na₂S₂O₃-Lösung versetzt, dreimal mit Ether extrahiert, und die vereinigten organischen Phasen werden getrocknet, eingeengt und über Kieselgel 60 (Petrolether / Essigester = 10/1) chromatographiert.
Ausbeute: 0,38 g (55% d.Th.)
R_{f} = 0,55 (Toluol)

### Herstellungsbeispiele

### Beispiel 1

### 4,6-Bis-(p-fluorphenyl)-5-hydroxymethyl-2-isopropyl-3-[(p-trifluormethylphenyl)-(hydroxy)-methyl]-pyridin

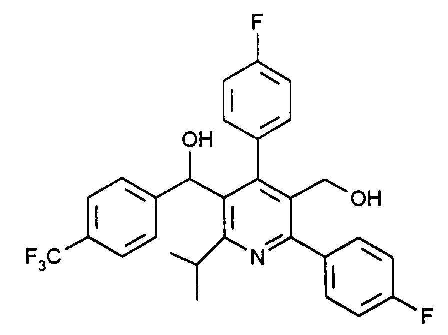

Zu 11 ml LiAlH₄ in THF (1 molare Lösung) wird unter Argon bei Reflux eine Lösung von 2 g (3,7 mmol) der Verbindung aus Beispiel V in 10 ml THF zugetropft und 1 h gerührt. Zur Aufarbeitung wird auf 0°C abgekühlt, mit 20%iger wäßriger Na-K-Tartratlösung versetzt, mit Wasser verdünnt und dreimal mit Essigester extrahiert. Die verreinigten organischen Phasen werden über Na₂SO₄ getrocknet, filtriert, eingeengt und das Produkt wird über Kieselgel 60 (Toluol/Essigester = 9/1) chromatographiert.
Ausbeute: 1,38 g (73% d.Th.)
R_{f} = 0,20 (Toluol/Essigester = 9/1)

### Beispiel 2

### 4,6-Bis-(p-fluorphenyl)-5-hydroxymethyl-2-isopropyl-3-p-trifluormethylbenzoylpyridin

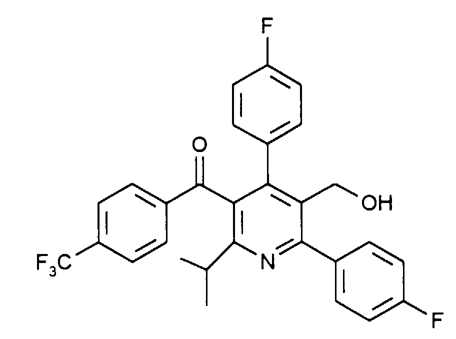

Zu einer Suspension von 50 mg (0,1 mmol) der Verbindung aus Beispiel XIV in 10 ml CH₃OH werden bei 0°C 4 mg (0,10 mmol) NaBH₄ zugegeben und nach Zusatz von 2 ml Dioxan wird 1 h gerührt. Zur Aufarbeitung wird mit 1 N AcOH angesäuert, dreimal mit Essigester extrahiert, und die vereinigten organischen Phasen werden über Na₂SO₄ getrocknet, filtriert und eingeengt.
Ausbeute: 48,3 mg (94% d.Th.)
R_{f} = 0,39 (CH₂Cl₂ / CH₃OH = 9/1)

### Beispiel 3

### 4,6-Bis-(p-flurophenyl)-2-isopropyl-3-[(p-trifluormethylphenyl)-(fluoro)-methyl]5-(1-hydroxyethyl)pyridin

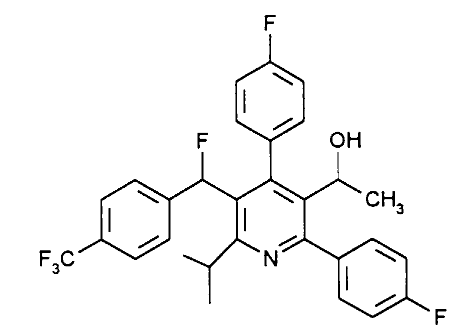

700 mg (1,36 mmol) der Verbindung aus Beispiel XVI werden unter Argon bei - 78°C mit 0,54 ml (3 M in THF, 1,63 mmol) Methylmagnesiumbromid in 10 ml THF 3 h gerührt. Nach Zusatz von weiteren 0,27 ml 3 M (0,81 mmol) CH₃MgBr und 1 h Rühren wird erwärmt, 1 h nachgerührt, mit gesättigter wäßriger NH₄Cl-Lösung und CH₂Cl₂ versetzt, auf pH 5 gebracht (1 N HCl), geschüttelt und getrennt. Die wäßrige Phase wird nochmals mit CH₂Cl₂ extrahiert, und die vereinigten organischen Phasen werden mit NaCl-Lösung geschüttelt, über Natriumsulfat getrocknet und eingeengt.
Ausbeute: 0,72 g (99,9% d.Th.)
R_{f} = 0,36 (Toluol)

### Beispiel 4

### 4,6-Bis-(p-fluorphenyl)-2-isopropyl-3-[(p-trifluormthylphenyl)-(fluoro)methyl]5-(3-hydroxy-1-propyl)pyridin

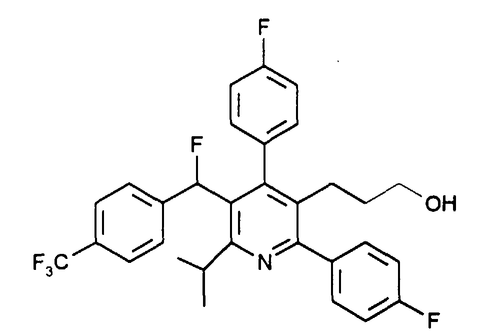

79 mg (0,14 mmol) der Verbindung aus Beispiel XVII werden in 4 ml THF unter Argon bei RT mit 0,37 ml 1 M (0,37 mmol) LiAlH₄-Lösung versetzt und 30 min gerührt. Anschließend wird in Eiswasser gegeben, mit 1 N HOAc auf pH 3 gestellt und dreimal mit Essigester extrahiert. Die vereinigten organischen Phasen werden über Natriumsulfat getrocknet, filtriert, eingeengt und über Kieselgel 60 (Petrolether / Essigester = 3/1) chromatographiert.
Ausbeute: 0,034 g (46% d.Th.)
R_{f} = 0,38 (Petrolether 40/60 : Essigester 3:1)

### Beispiel 5

### 4,6-Bis-(p-fluorphenyl)-2-isopropyl-3-p-trifluormethylbenzoyl-5-(2-hydroxy-1-ethyl)-pyridin

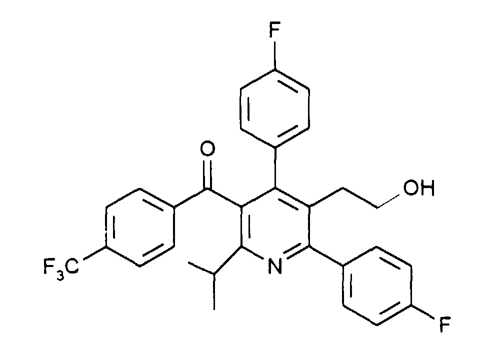

0,05 g (0,1 mmol) der Verbindung aus Beispiel XIX werden in 20 ml EtOH suspendiert, mit 8 mg (0,2 mmol) NaBH₄ versetzt und 2 h bei RT gerührt. Anschließend wird mit Wasser versetzt, auf pH 5 gestellt (1 N AcOH) und dreimal mit Essigester extrahiert. Die vereinigten organischen Phasen werden getrocknet, eingeengt und über Kieselgel 60 (Petrolether / Essigester = 3/1) chromatographiert.
Ausbeute: 0,03 g (65% d.Th.)
R_{f} = 0,79 ( Petrolether / Essigester = 2/1)

In Analogie zu den Vorschriften der Beispiele 1-5 werden die in den Tabellen 1-4 aufgeführten Verbindungen hergestellt:

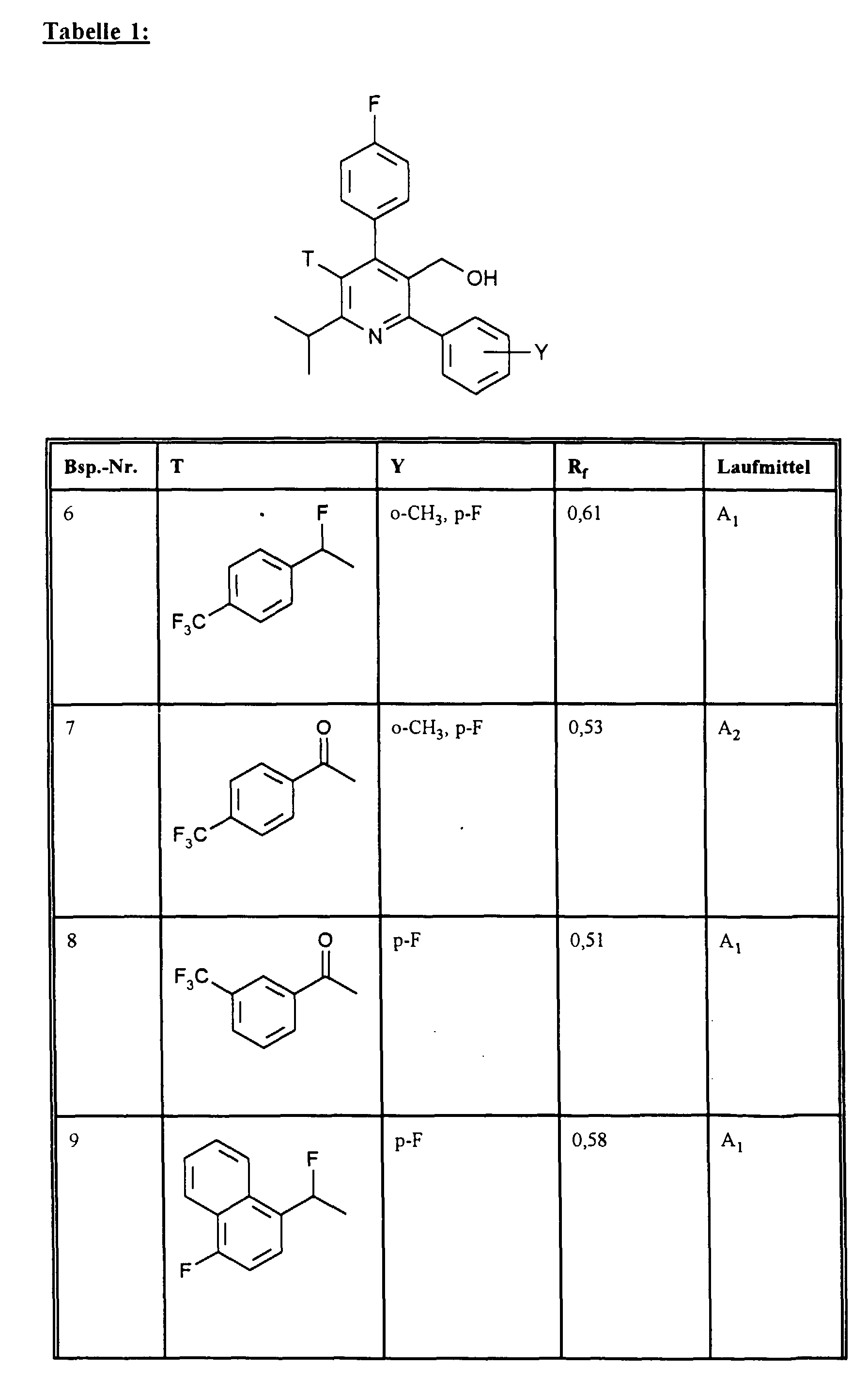

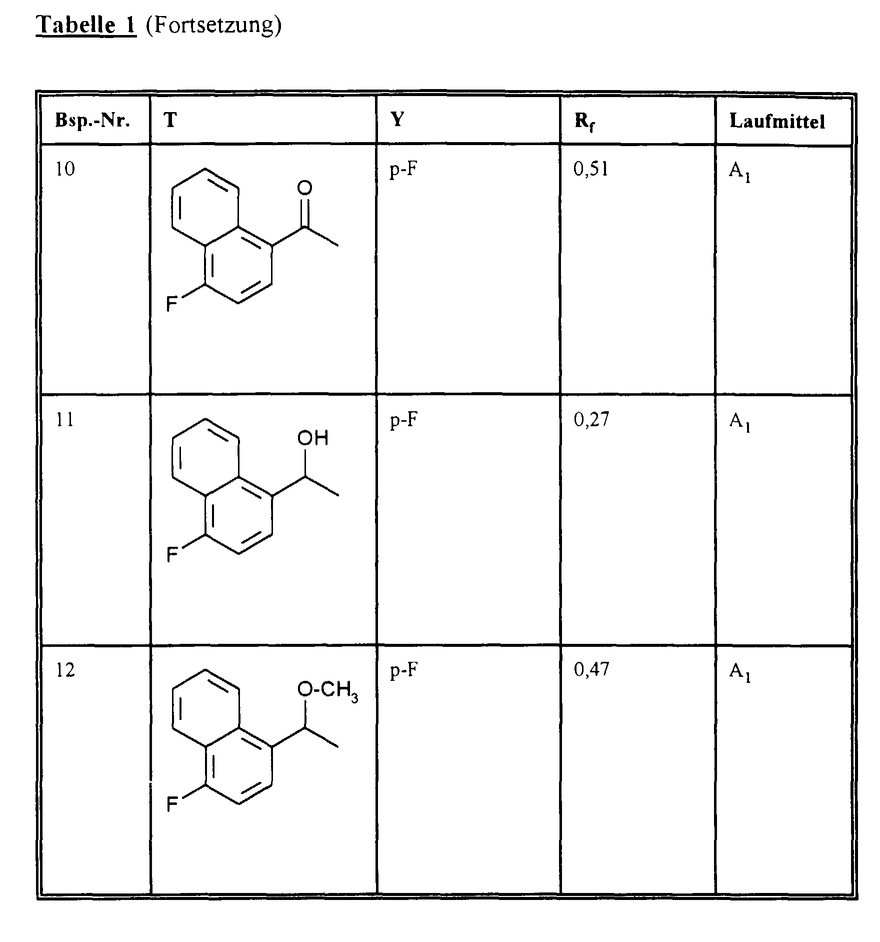

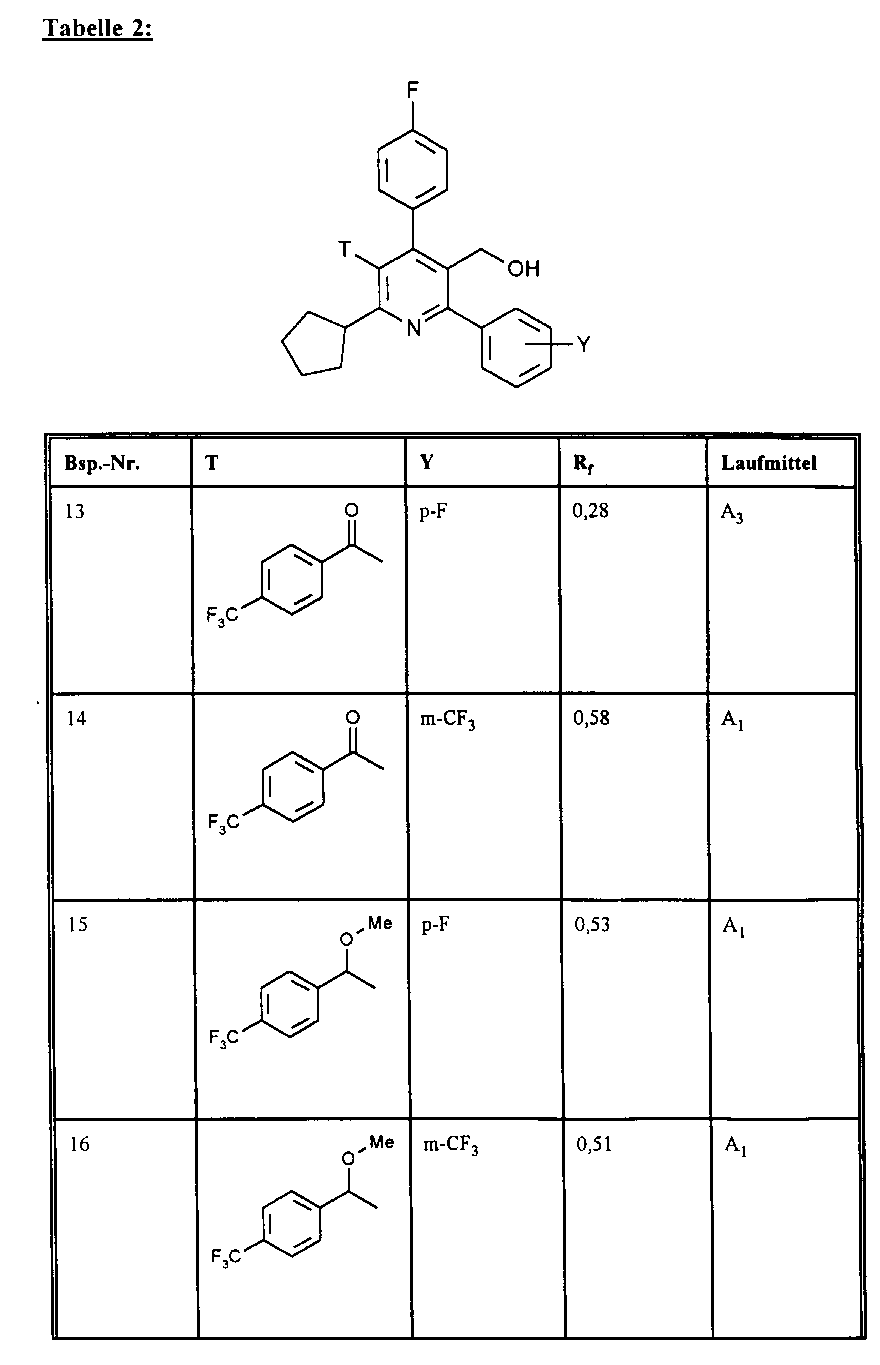

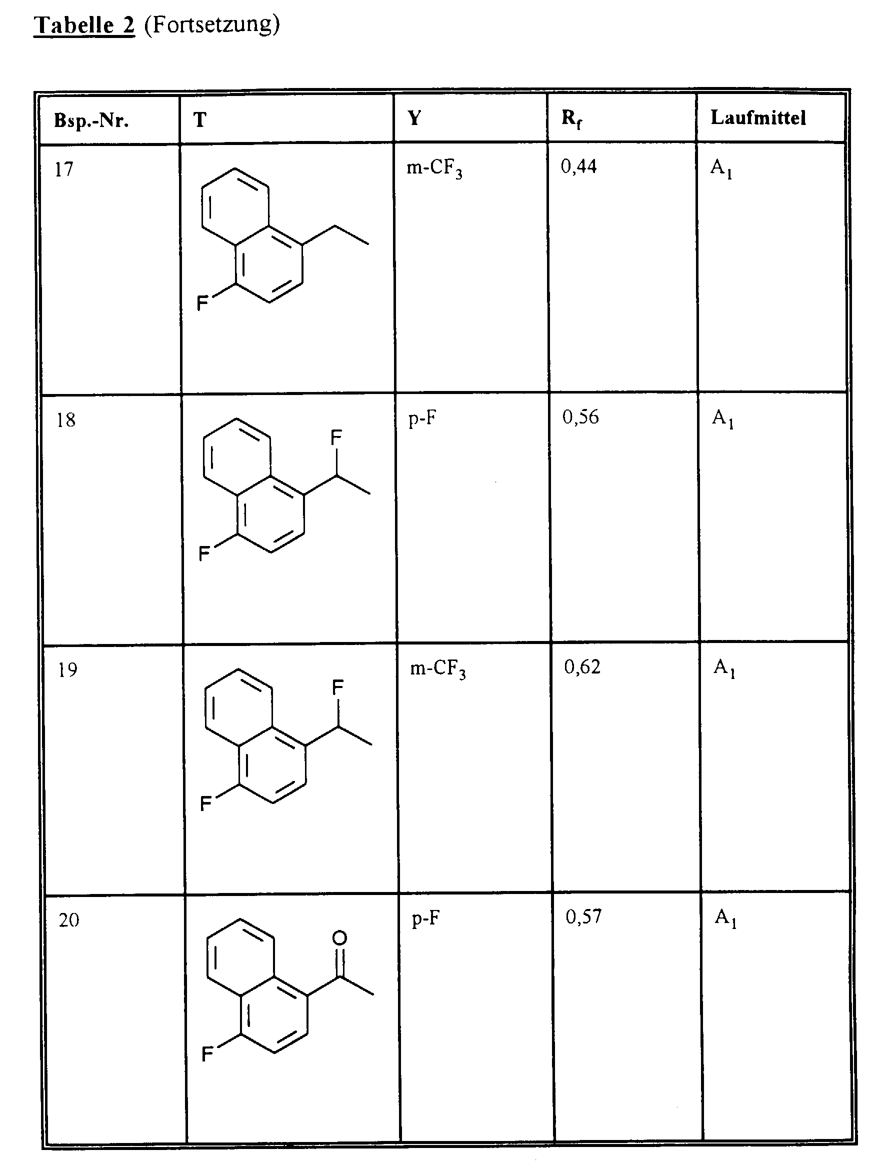

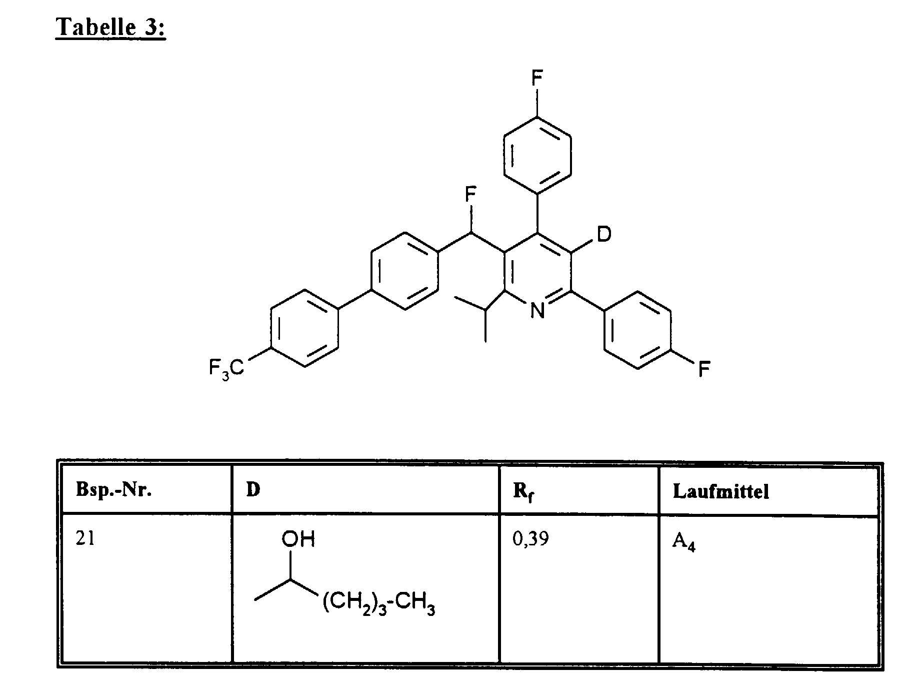

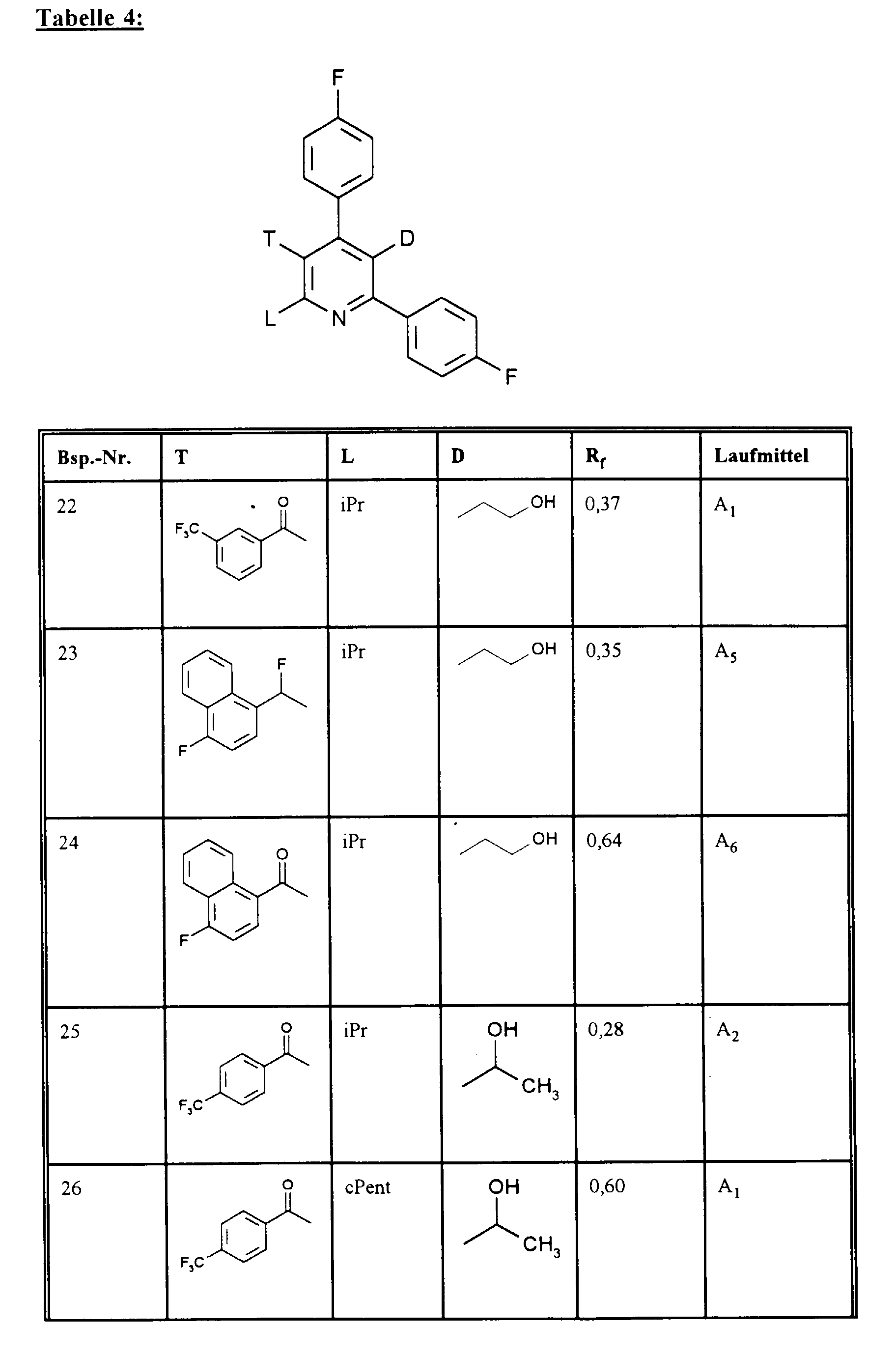

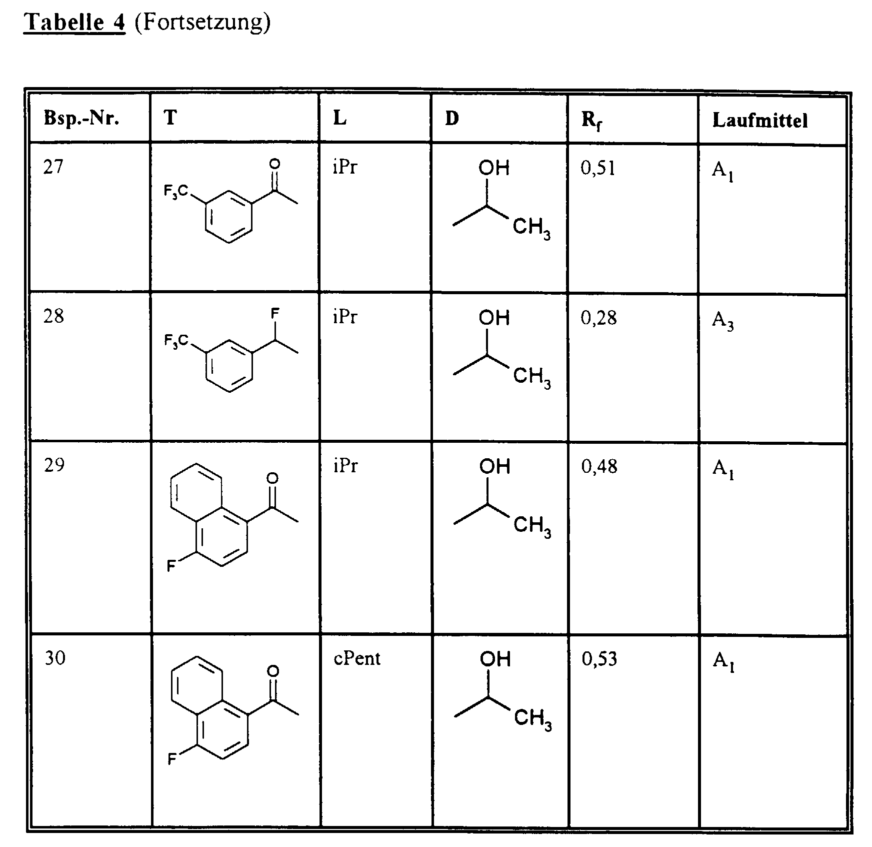

### Beispiel 31

### 2,4-Bis-(4-fluorphenyl)-6-isopropyl-5-[4-(trifluormethylphenyl)-fluormethyl]-3-hydroxymethyl-pyridin

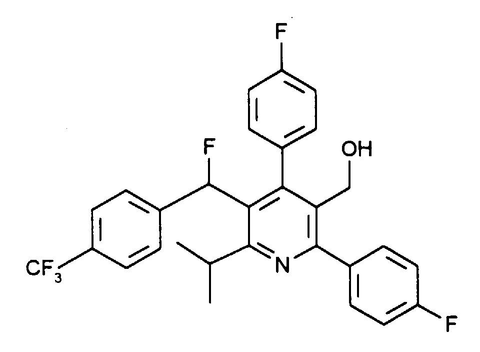

Unter Argon erwärmt man eine Mischung von 23,04 mg (0,607 mmol; 1,1 eq.) LiAlHH₄ und 5 ml THF p.a. auf 60°C bis 80°C und läßt 0,3 g (0,552 mmol) der Verbindung aus Beispiel VI gelöst in 5 ml THF zutropfen. Danach wird 1 h unter Rückfluß gekocht. Nach Abkühlen auf 0°C gibt man 5 ml einer 20%igen Kaliumnatriumtartrat-Lösung und 10 ml Wasser hinzu und extrahiert zweimal mit je 80 ml Essigester. Die vereinigten organischen Phasen werden einmal mit gesättigter NaCl-Lösung gewaschen, über Na₂SO₄ getrocknet und im Vakuum eingeengt. Der Rückstand wird an 60 g Kieselgel 60 zunächst mit Toluol und anschließend mit Toluol / Essigester (9:1) chromatographiert.
Ausbeute: 156 mg (54,9% d.Th.)
R_{f} = 0,53 (Toluol / Essigester 9:1)

### Beispiel 32

### 2,4-Bis-(4-fluorphenyl)-6-isopropyl-5-(4-trifluormethylbenzyl)-3-hydroxymethyl-pyridin

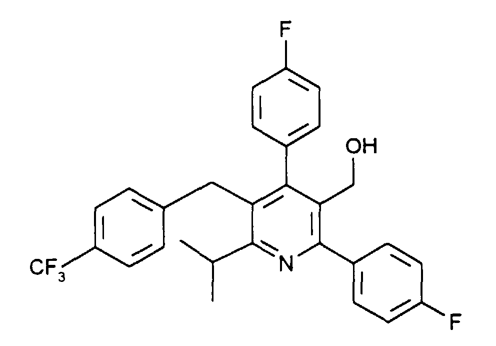

11,2 mg (0,295 mmol; 3,0 eq.) LiAlH₄ werden unter Argon in 2 ml THF p.a. bei 80°C suspendiert. 55 mg (0,0982 mmol) der Verbindung aus Beispiel VII in 2 ml THF p.a. werden bei 80°C zugetropft. Nach 8 h Rühren bei 80°C wird die Lösung auf 20°C gekühlt, mit 5 ml 20%iger Kaliumnatriumtartrat-Lösung versetzt, zweimal mit 20 ml Essigester extrahiert, über Na₂SO₄ getrocknet und die organische Phase eingeengt. Der Rückstand wird durch Kieselgelchromatographie mit Toluol gereinigt.
Ausbeute: 36 mg (73,0% d.Th.)
R_{f} = 0,58 (Toluol / Essigester 9:1)

### Beispiel 33

### 2,4-Bis-(4-fluorphenyl)-6-isopropyl-5-[4-(trifluormethylphenyl)-aminomethyl]-3-hydroxymethyl-pyridin

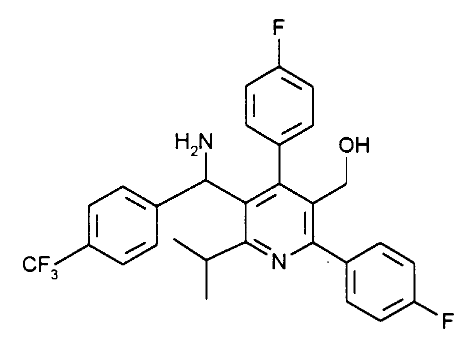

90 mg (0,167 mmol) der Verbindung aus Beispiel IX werden unter Argon in 5 ml Toluol p.a. bei -78°C gelöst und 0,84 ml (1 mmol; 6 eq.) Diisobutylaluminiumhydrid (DIBAL-H; 1,2 molar in Toluol) aus einer Spritze zugegeben. Man rührt noch 15 min bei -78°C und lagert danach die Reaktionslösung bei -30°C über Nacht. Anschließend kühlt man auf -78°C ab, gibt 2 ml 20%ige Kaliumnatriumtartrat-Lösung hinzu und verdünnt mit Toluol. Die Lösung wird einmal mit gesättigter NaCl-Lösung gewaschen, über Na₂SO₄ getrocknet und eingeengt. Der Rückstand wird durch Chromatographie an Kieselgel (40 g) mit Toluol und Toluol/Essigester (8:2) gereinigt.
Ausbeute: 60 mg (70,3% d.Th.)
R_{f} = 0,27 (A₁₁)

### Beispiel 34

### 2,4-Bis-(4-fluorphenyl)-6-cyclopentyl-5-[2-(benzothiazol-2-yl)-fluormethyl]-3-hydroxymethyl-pyridin

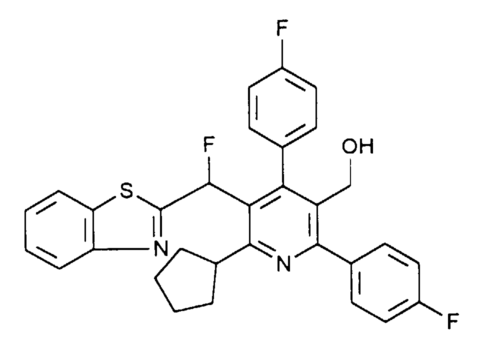

Nach Beispiel 31 werden 450 mg (0,9 mmol) der Verbindung aus Beispiel XI mit 44,4 mg (1,171 mmol; 1,3 eq) LiAlH₄ in 30 ml THF zum Alkohol reduziert.
Ausbeute: 182 mg (42,6% d.Th.)
R_{f} = 0,45 (Toluol / Essigester 9:1)

Nach den oben angegebenen Vorschriften werden die in den Tabellen 5 - 8 aufgeführten Verbindungen hergestellt:

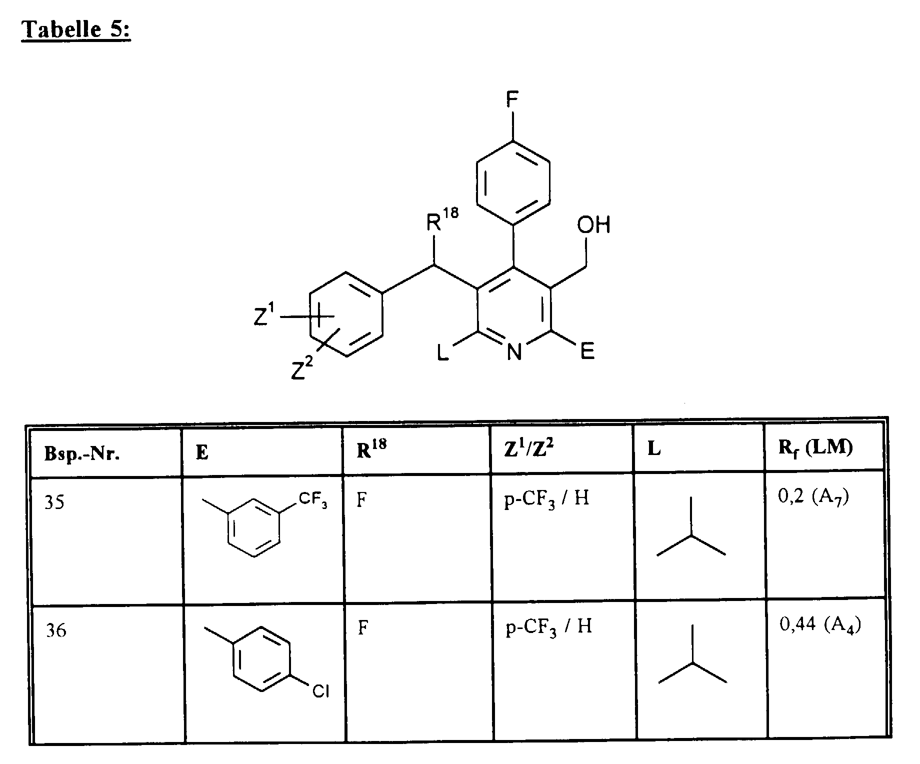

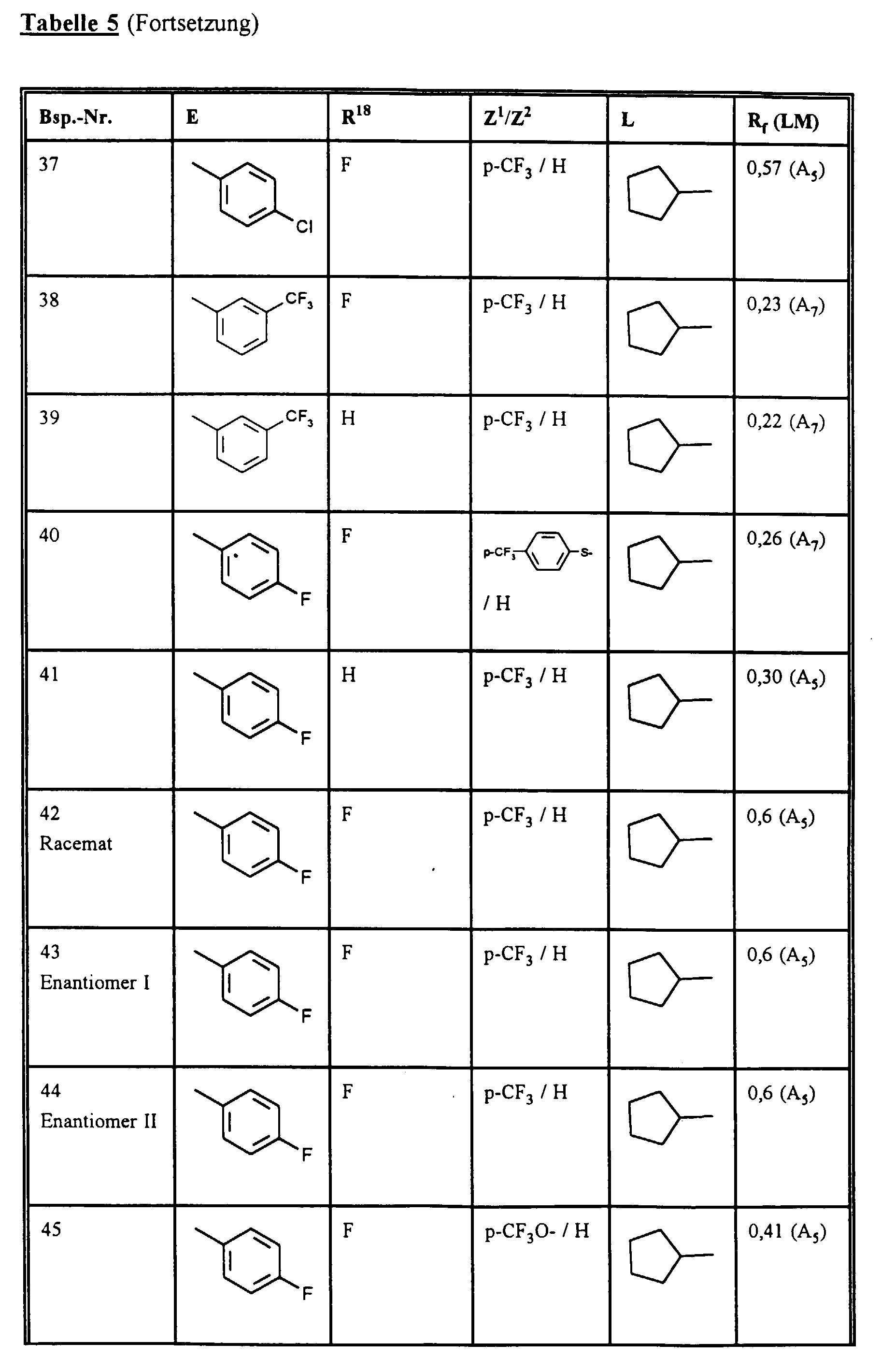

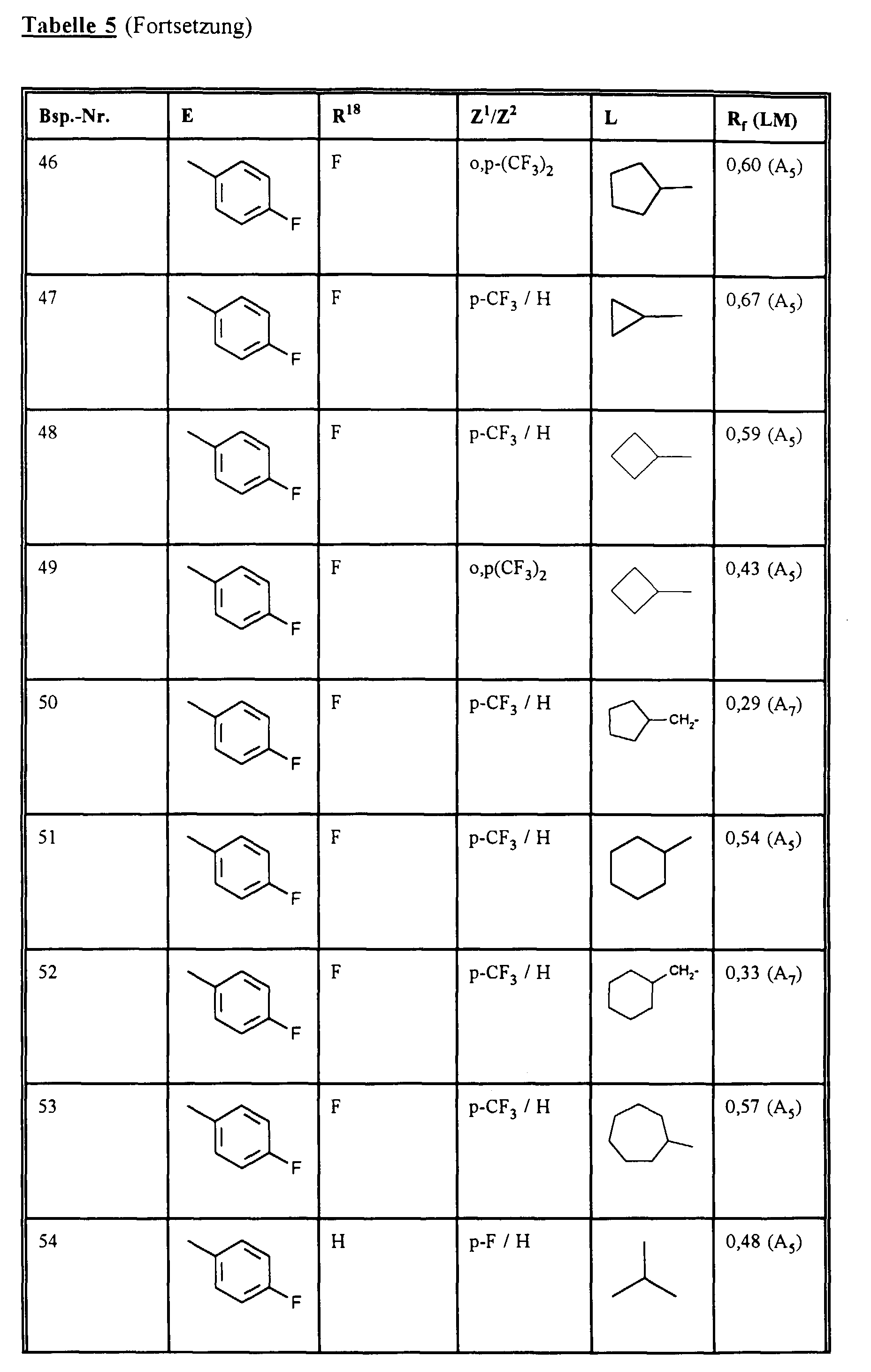

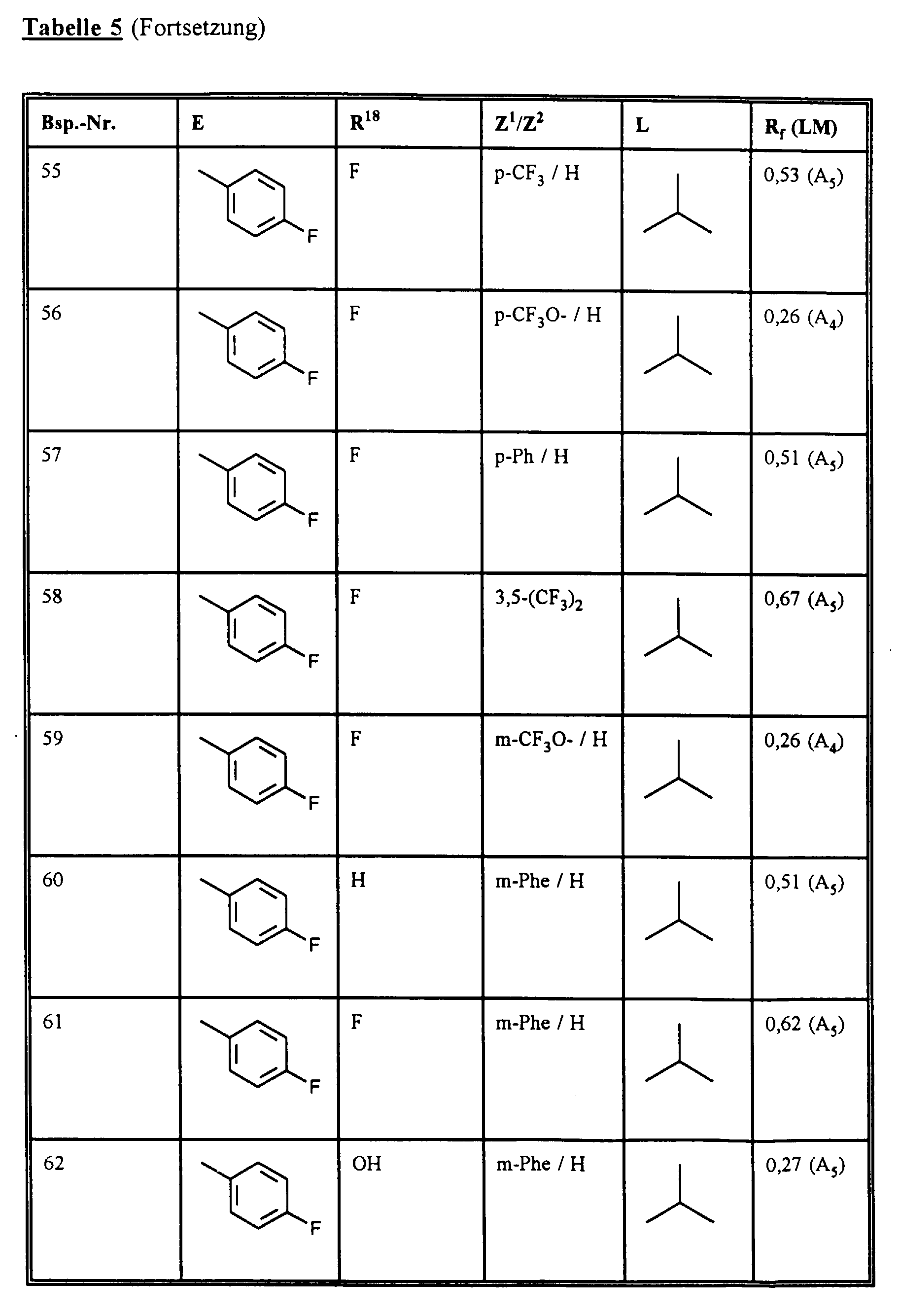

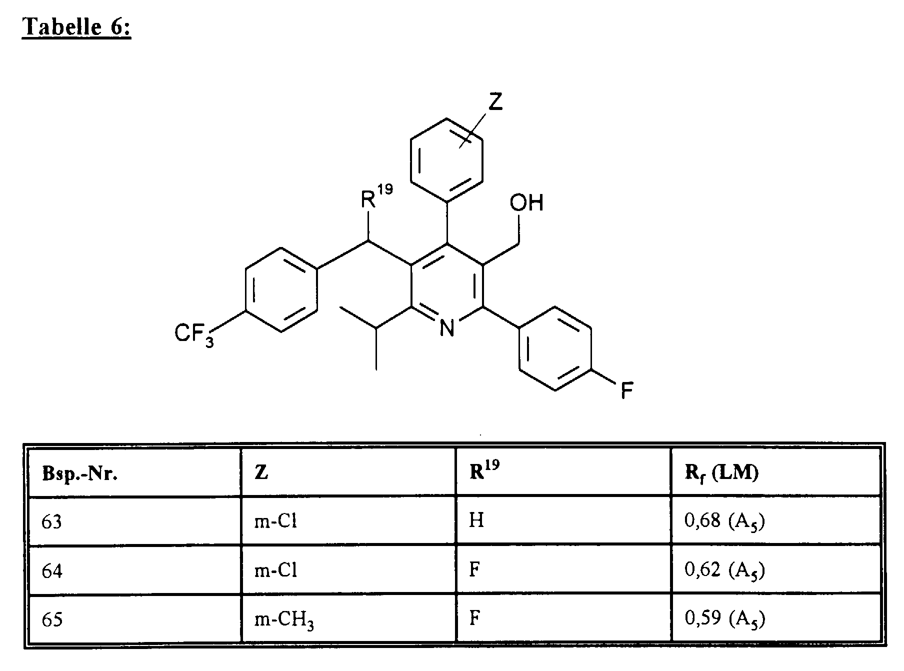

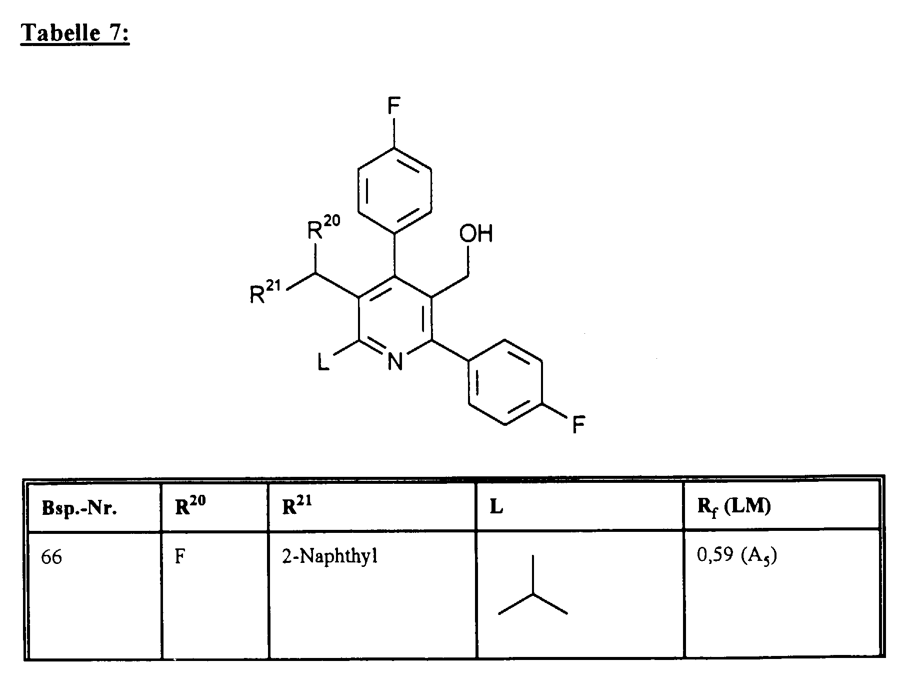

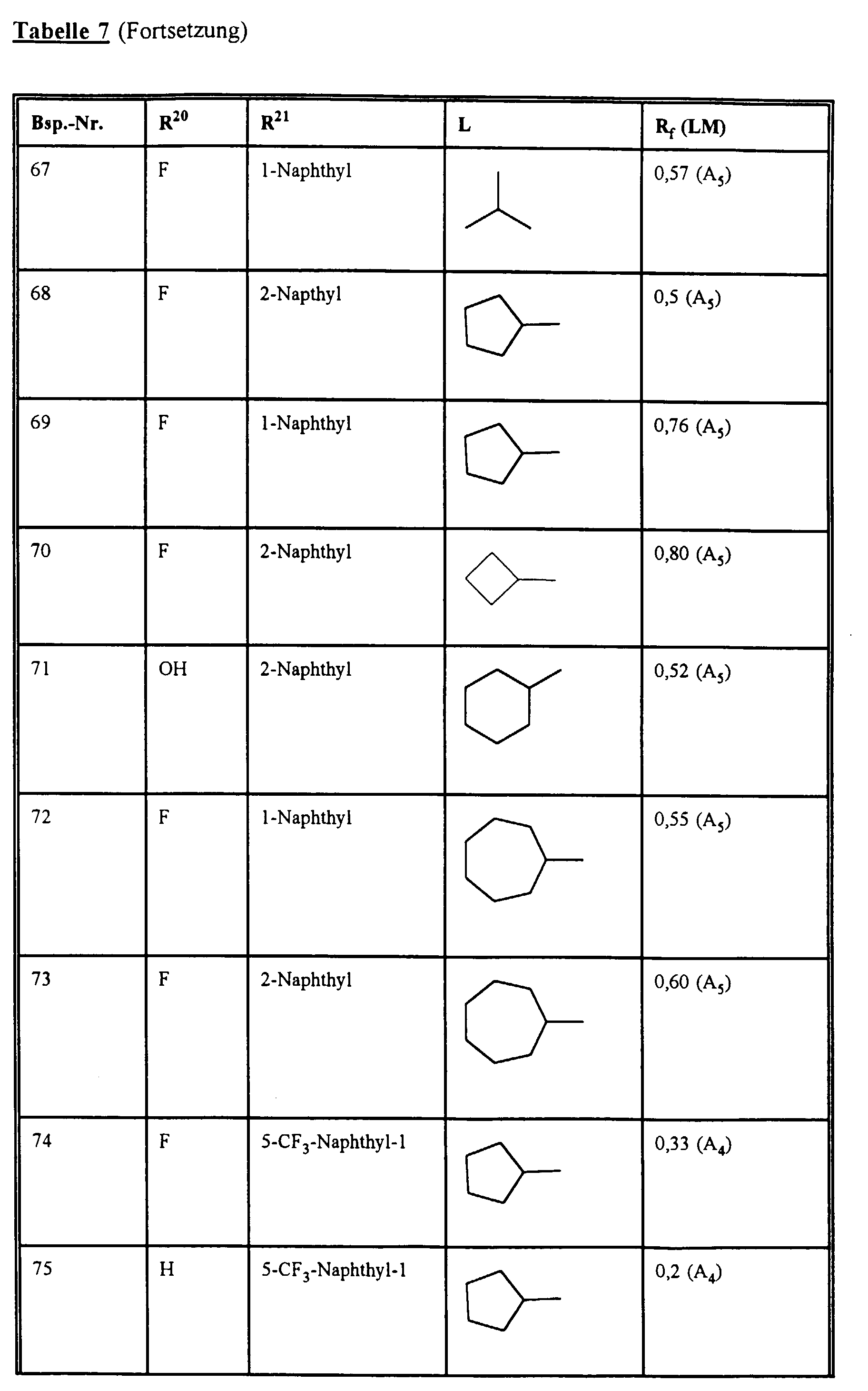

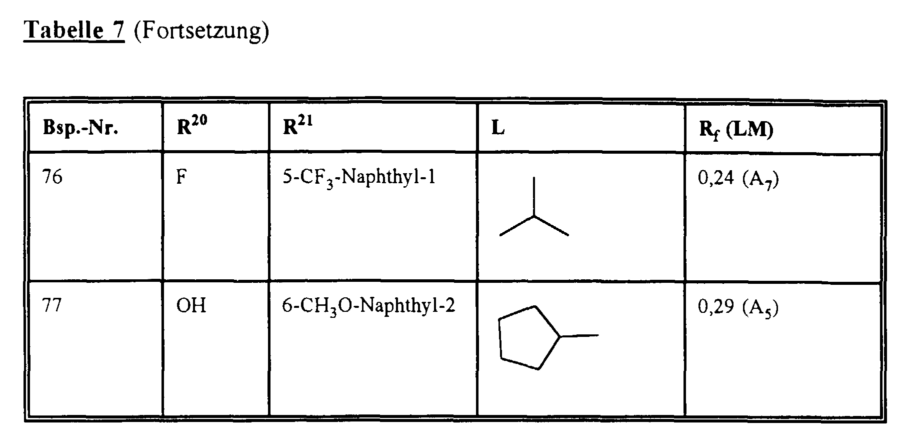

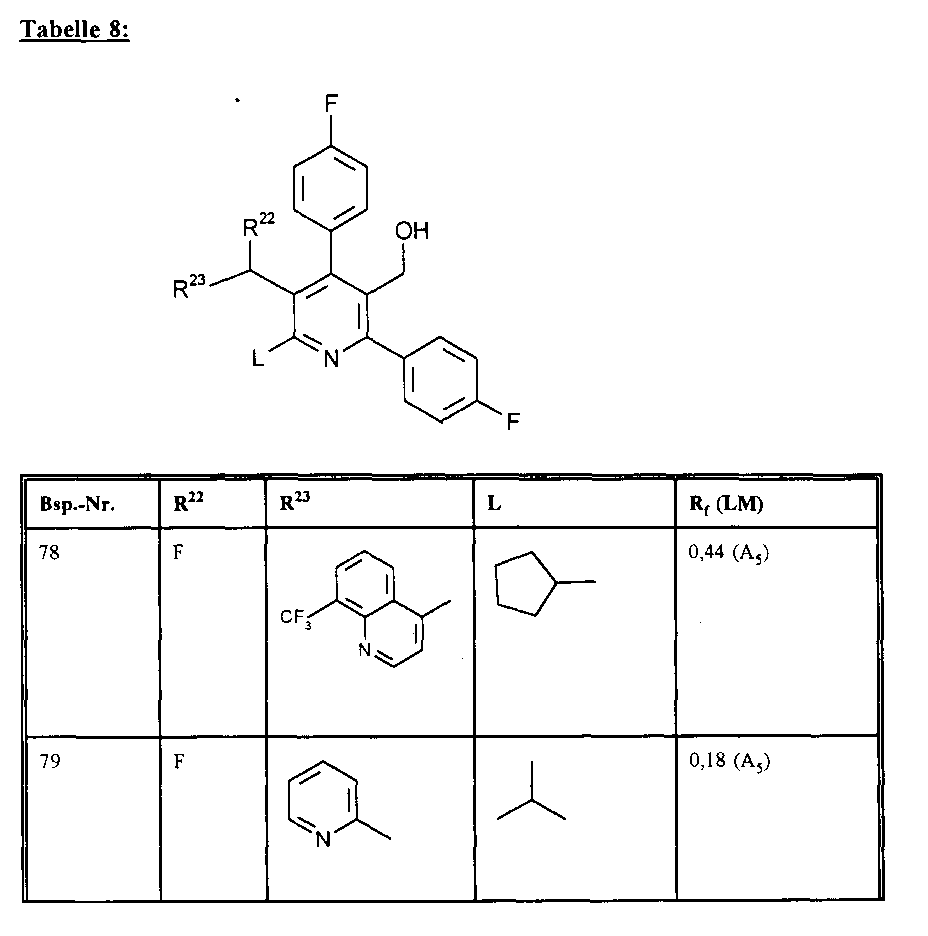

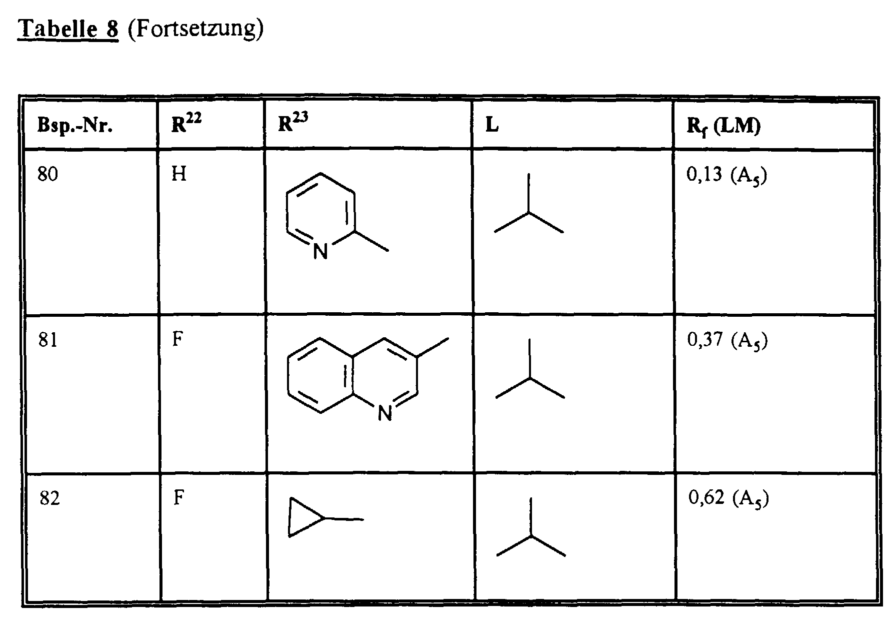

### Beispiel 83

### 2,4-Bis-(4-fluorphenyl)-6-isopropyl-5-[2-(3-trifluormethyl-phenyl)vinyl]-3-hydroxymethyl-pyridin

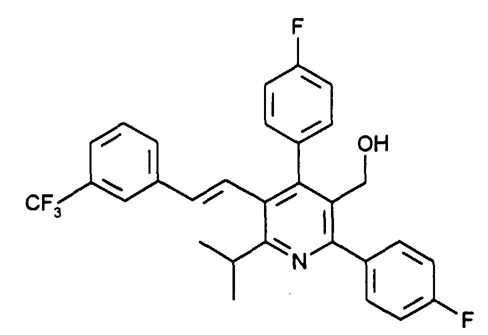

Unter Argon gibt man zu einer Lösung von 269 mg (0,5 mmol) der Verbindung aus Beispiel XII in 10 g trockenem Toluol bei -78°C 3,0 ml (3 mmol; 6 eq.) DIBAL-H hinzu und rührt danach ohne Kältebad 4 h bis auf +15°C. 40 ml Essigester und 20 ml einer 20%igen Kaliumnatriumtartrat-Lösung fügt man hinzu und rührt 10 min. Die wäßrige Phase wird abgetrennt, die organische Schicht über Na₂SO₄ getrocknet, filtriert und eingeengt.
Ausbeute: 250 mg (98% d.Th.)
R_{f} = 0,38 (A₄)

### Beispiel 84

### 2,4-Bis-(4-fluorphenyl)-6-isopropyl-5-[2-(3-trifluormethyl-phenyl)ethyl]-3-hydroxymethyl-pyridin

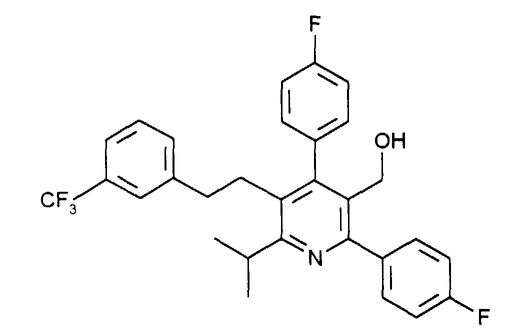

100 mg (0,196 mmol) der Verbindung aus Beispiel 83 werden in 20 g Methanol in Gegenwart von 100 mg Pd/C (10%) unter Wasserstoffatmosphäre bei Raumtemperatur über Nacht gerührt. Danach wird der Katalysator über SiO₂ abgesaugt, mit Methanol nachgewaschen und das Filtrat eingeengt. Der Rückstand wird über 30 ml Kieselgel durch Flash-Chromatographie mit Toluol gereinigt.
Ausbeute: 71 mg (70,1% d.Th.)
R_{f} = 0,25 (A₄)

### Beispiel 85

### 2,4-Bis-(4-fluorphenyl)-6-isopropyl-5-[2-(3-trifluormethyl-phenyl)-1,2-dihydroxyethyl]-3-hydroxymethyl-pyridin

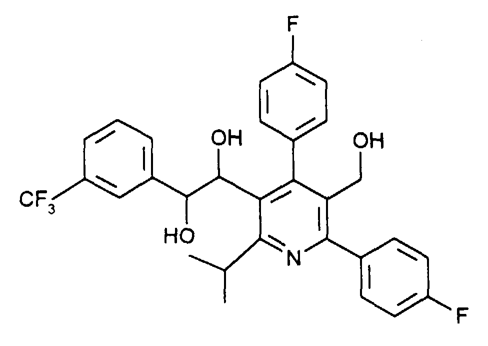

Analog Beispiel 33 werden 76 mg (0,133 mmol) der Verbindung aus Beispiel XIII mit 0,33 ml (0,333 mmol, 2,5 eq.) DIBAL-H (1 molar in Toluol) umgesetzt.
Ausbeute: 31 mg (43% d.Th.)
R_{f} = 0,16 (Toluol / Essigester 8:2)

Nach diesen Vorschriften werden die in den Tabellen 9 und 10 aufgeführten Verbindungen hergestellt:

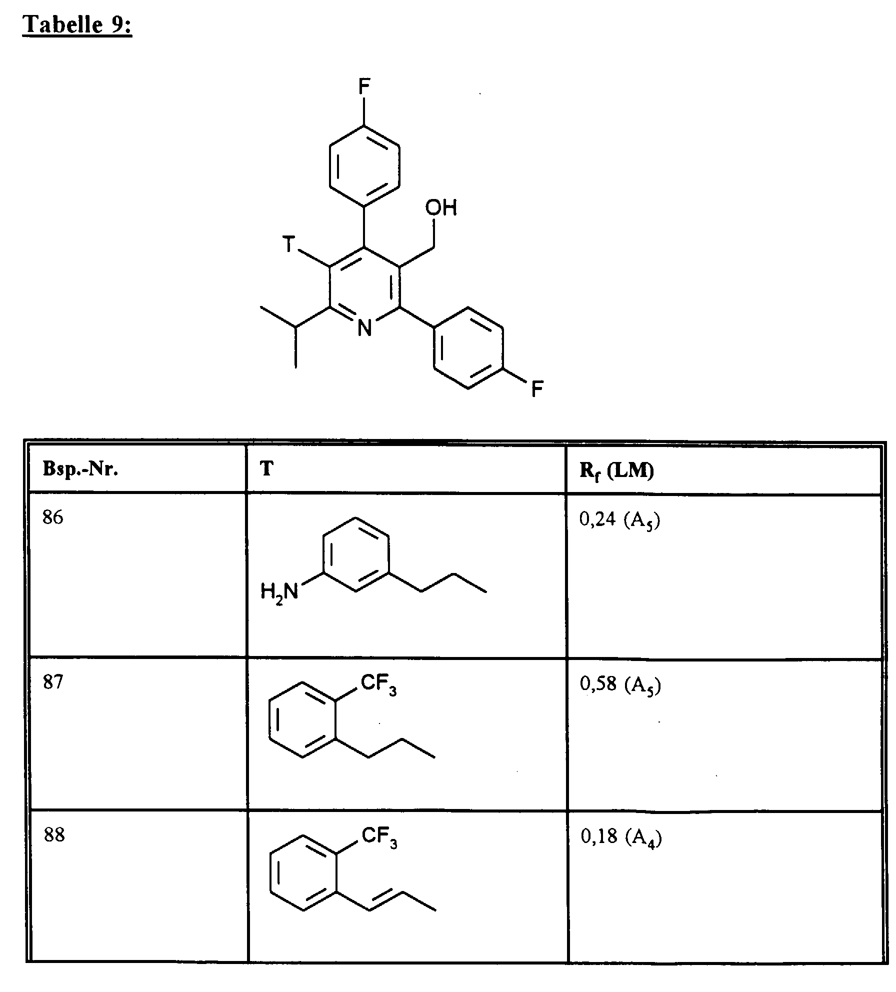

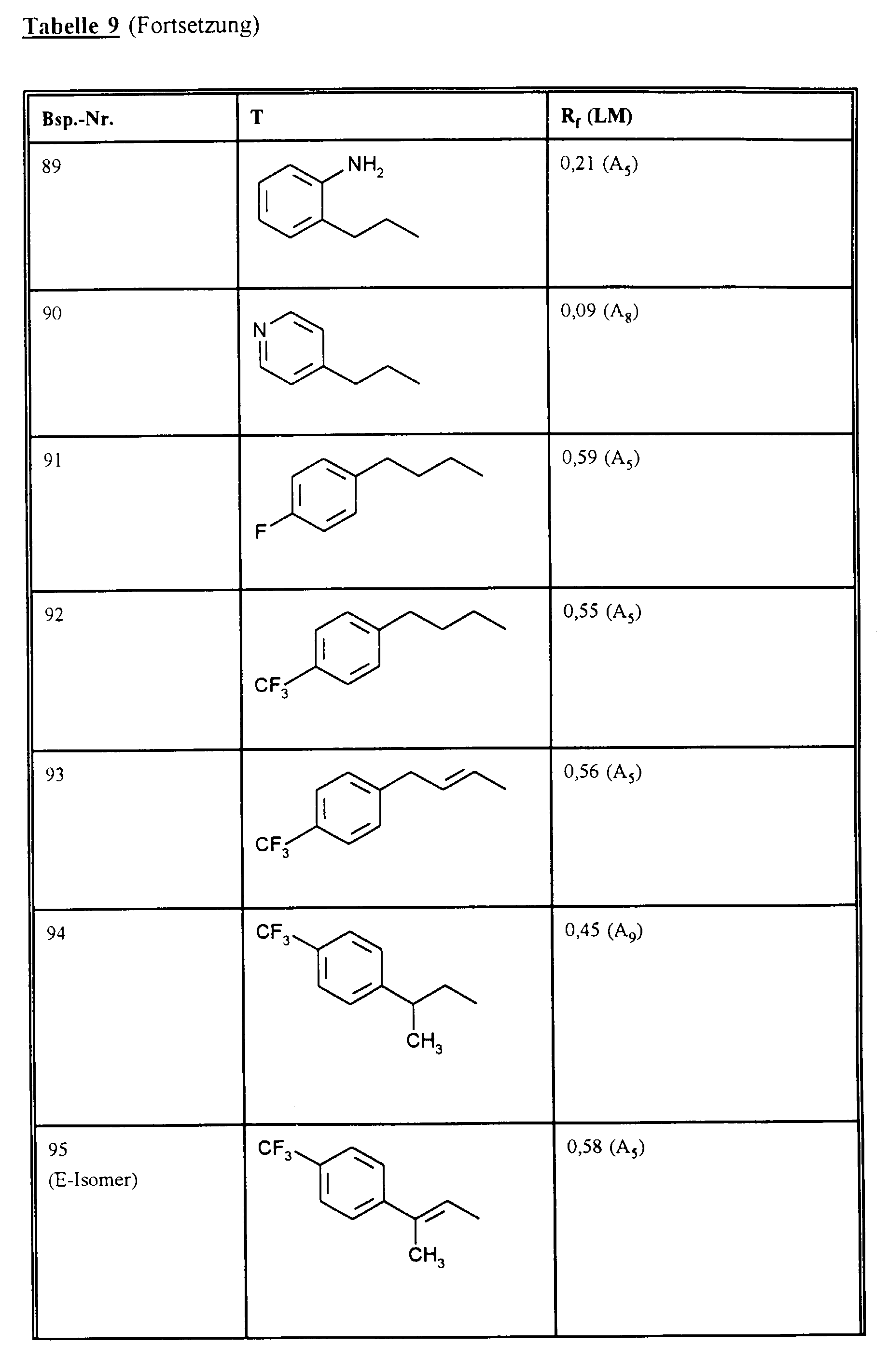

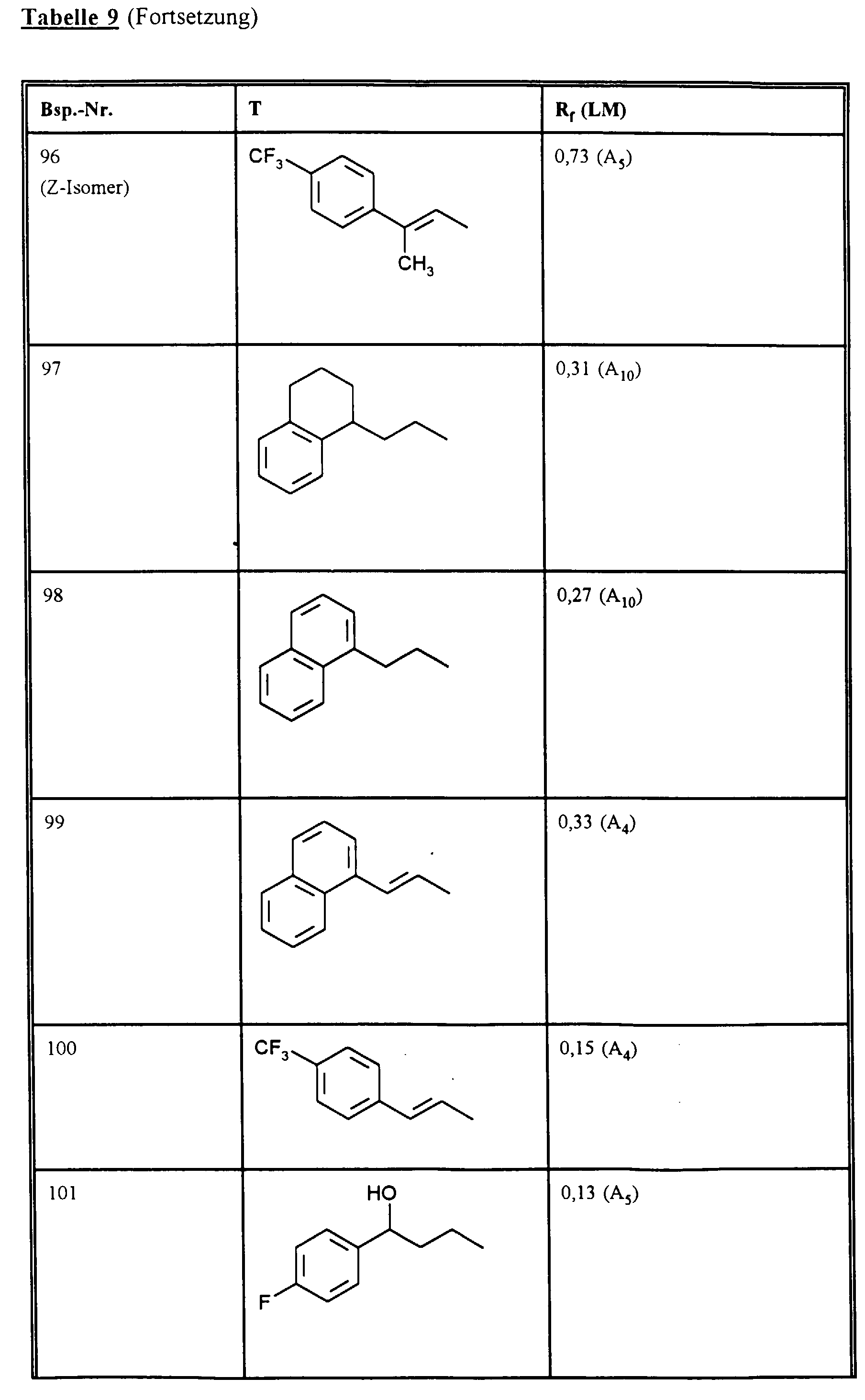

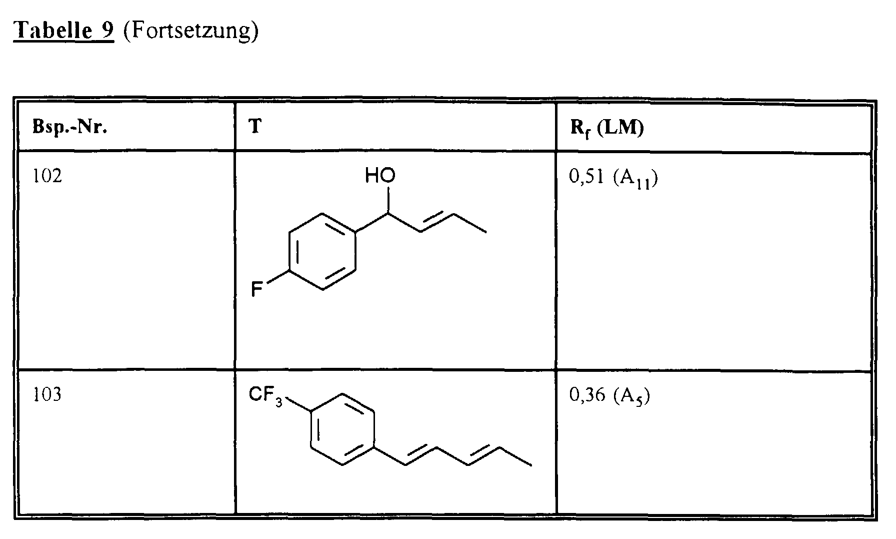

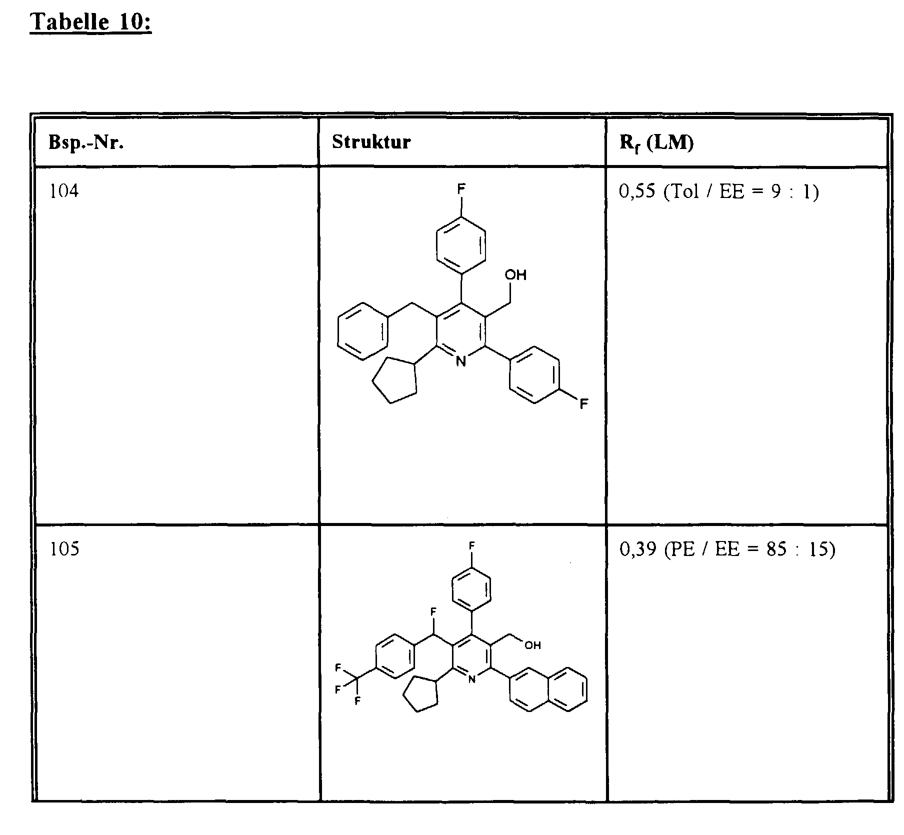

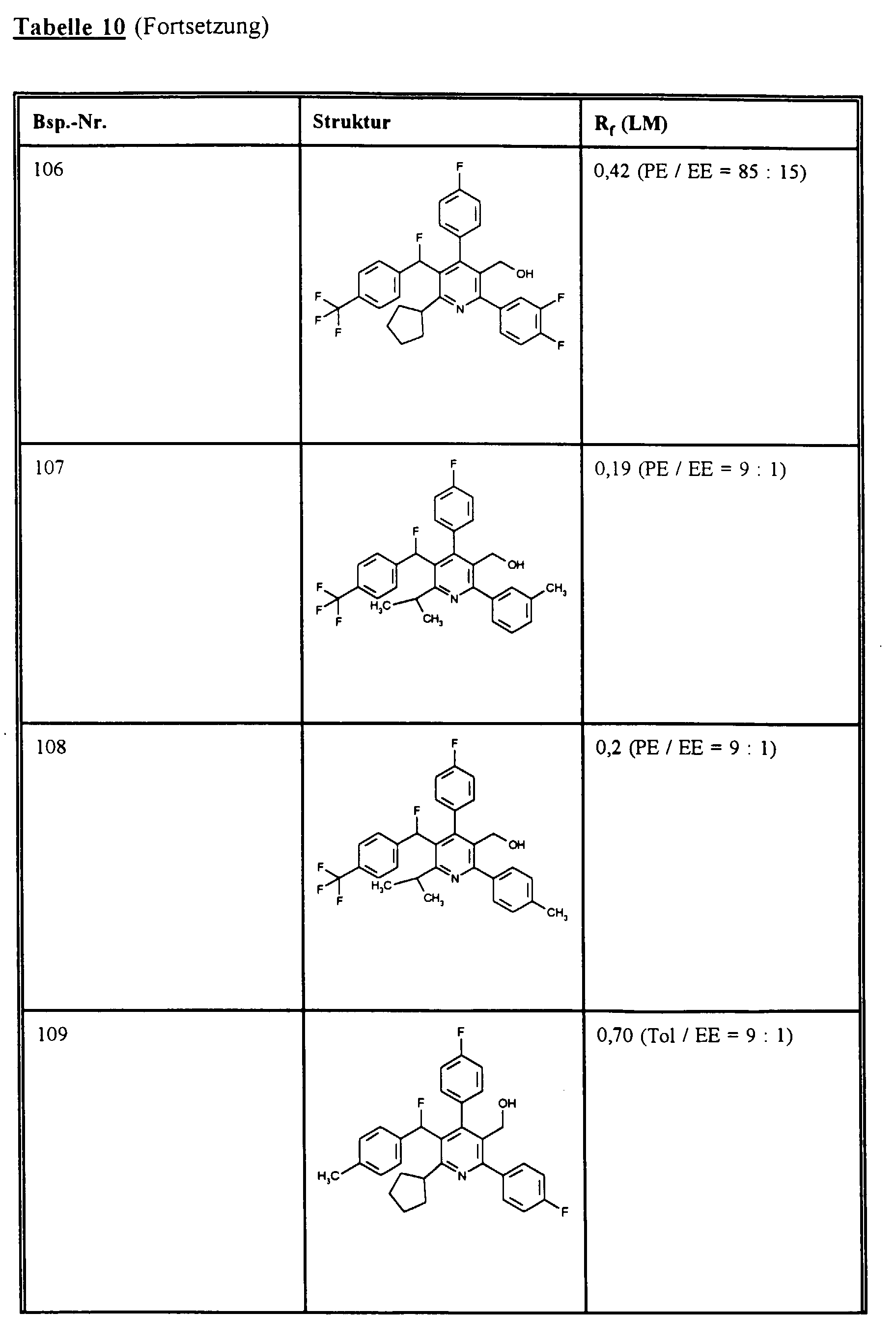

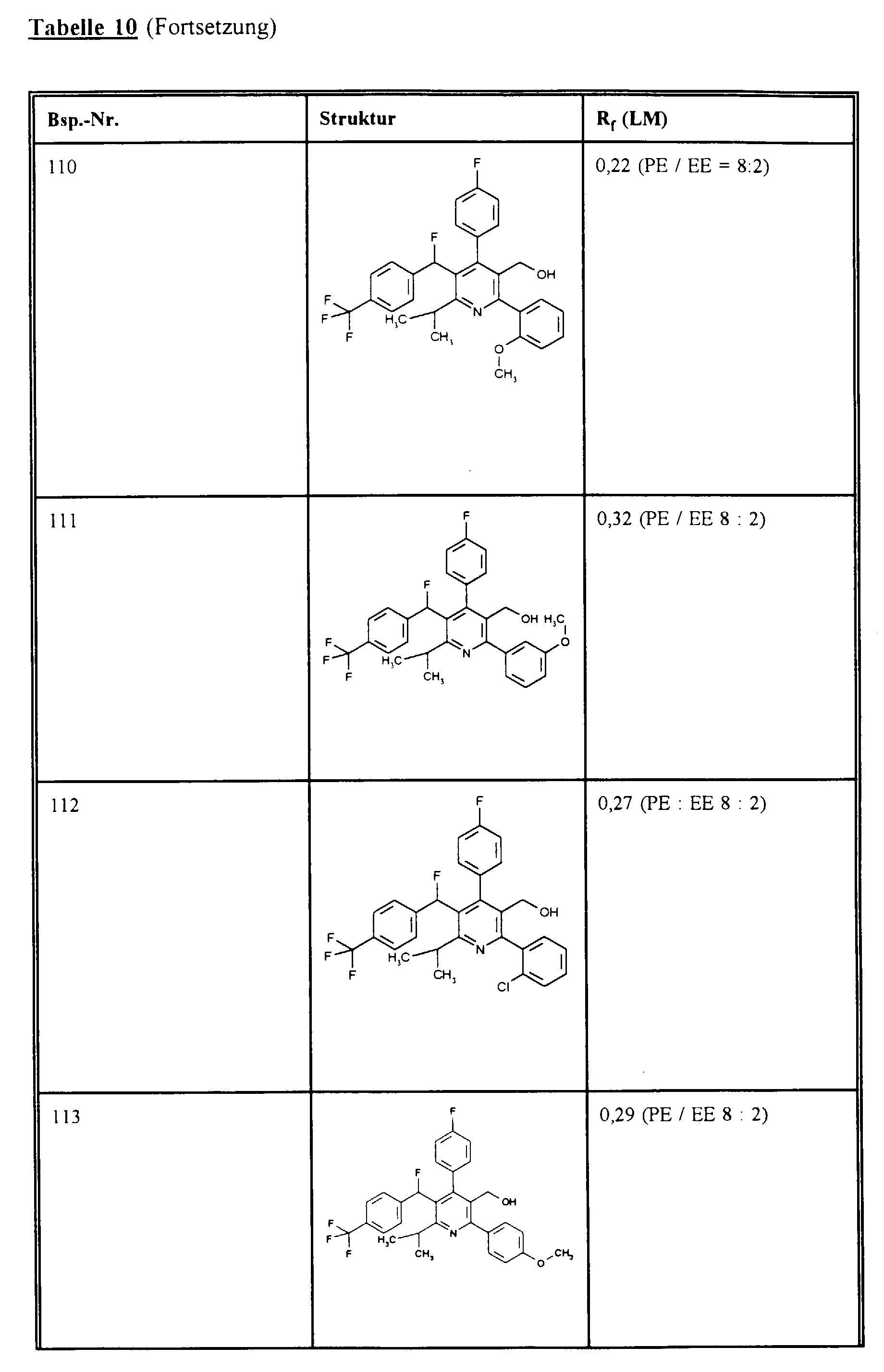

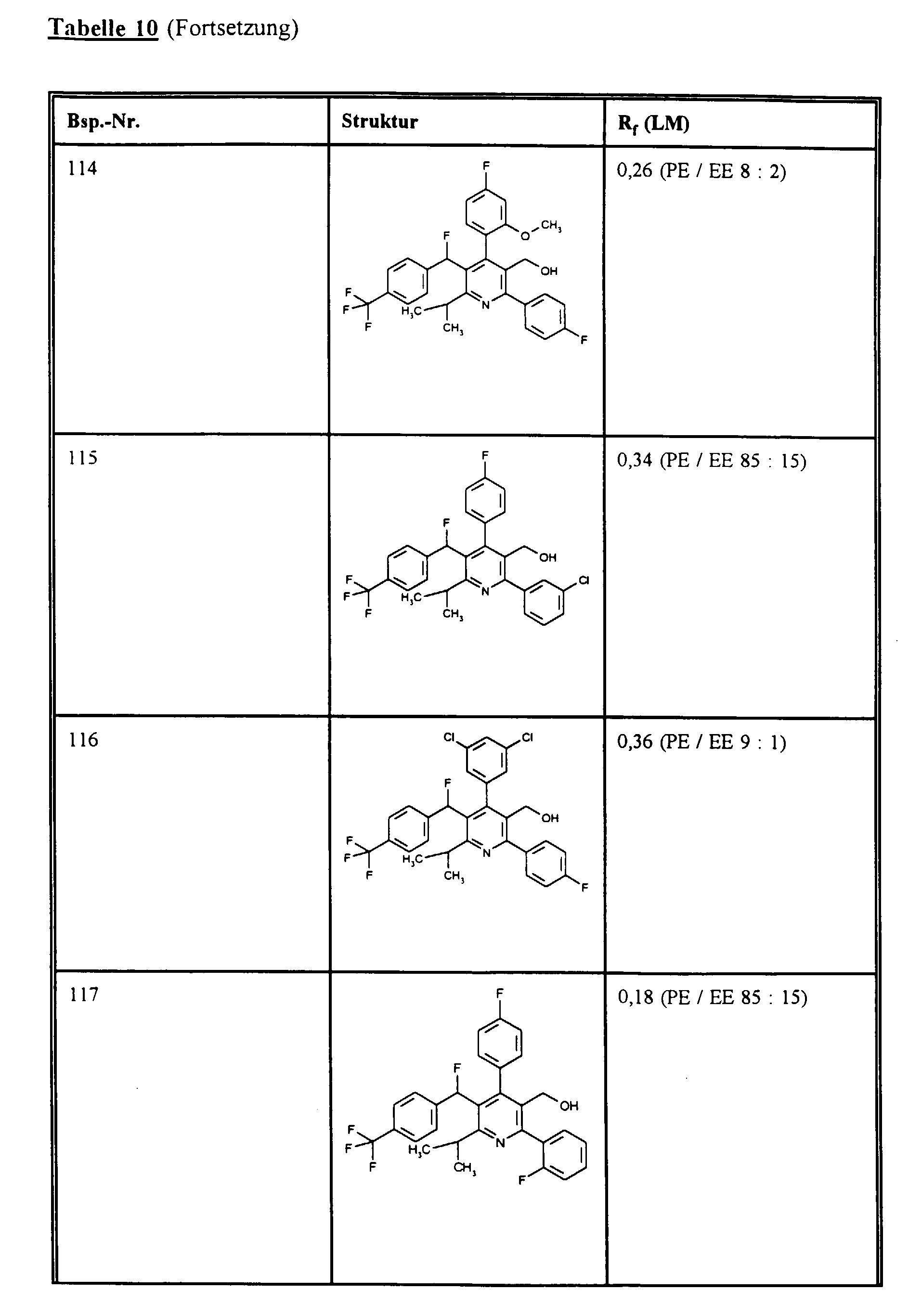

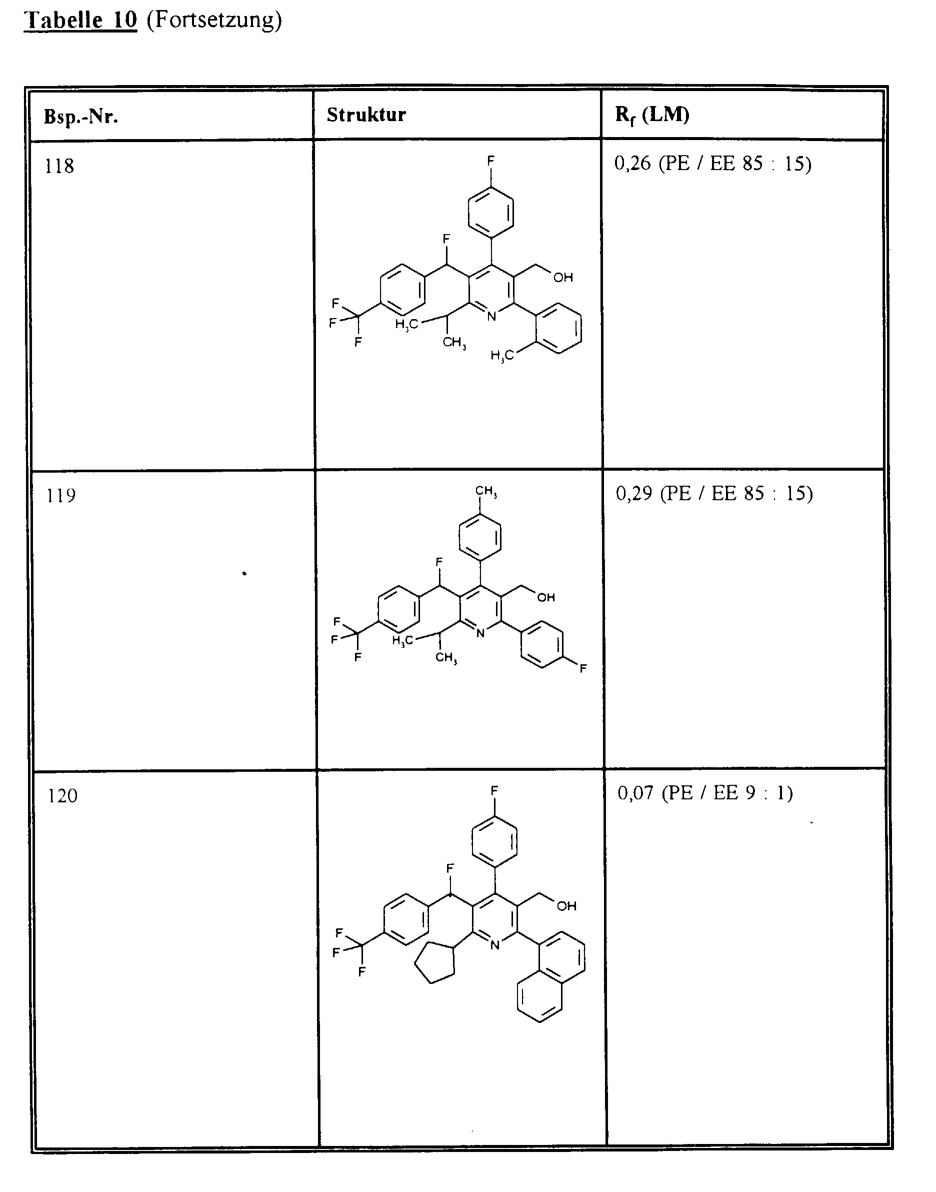

## Patentansprüche

1. 2-Aryl-substituierte Pyridine der allgemeinen Formel (I) 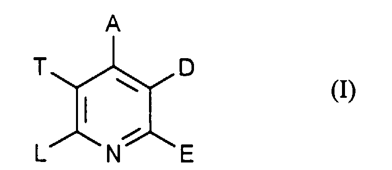 in welcher
A und E gleich oder verschieden sind und für Aryl mit 6 bis 10 Kohlenstoffatomen stehen, das gegebenenfalls bis zu 5-fach gleich oder verschieden durch Halogen, Hydroxy, Trifluormethyl, Trifluormethoxy, Nitro oder durch geradkettiges oder verzweigtes Alkyl, Acyl, Hydroxyalkyl oder Alkoxy mit jeweils bis zu 7 Kohlenstoffatomen, oder durch eine Gruppe der Formel -NR¹R² substituiert ist,
worin
R¹ und R² gleich oder verschieden sind und Wasserstoff, Phenyl oder geradkettiges oder verzweigtes Alkyl mit bis zu 6 Kohlenstoffatomen bedeuten,
D für geradkettiges oder verzweigtes Alkyl mit bis zu 8 Kohlenstoffatomen steht, das durch Hydroxy substituiert ist,
L für Cycloalkyl mit 3 bis 8 Kohlenstoffatomen steht, oder für geradkettiges oder verzweigtes Alkyl mit bis zu 8 Kohlenstoffatomen steht, das gegebenenfalls durch Cycloalkyl mit 3 bis 8 Kohlenstoffatomen oder durch Hydroxy substituiert ist,
T für einen Rest der Formel
R³―X―
oder 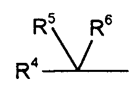 steht,
worin
R³ und R⁴ gleich oder verschieden sind und Cycloalkyl mit 3 bis 8 Kohlenstoffatomen bedeuten, oder
Aryl mit 6 bis 10 Kohlenstoffatomen, oder einen 5- bis 7-gliedrigen aromatischen, gegebenenfalls benzokondensierten Heterocyclus mit bis zu 3 Heteroatomen aus der Reihe S, N und/oder O bedeuten, die gegebenenfalls bis zu 3-fach gleich oder verschieden durch Trifluormethyl, Trifluormethoxy, Halogen, Hydroxy, Carboxyl, Nitro, durch geradkettiges oder verzweigtes Alkyl, Acyl, Alkoxy oder Alkoxycarbonyl mit jeweils bis zu 6 Kohlenstoffatomen oder durch Phenyl, Phenoxy oder Phenylthio substituiert sind, die ihrerseits durch Halogen, Trifluormethyl oder Trifluormethoxy substituiert sein können,
und/oder die Cyclen gegebenenfalls durch eine Gruppe der Formel -NR⁷R⁸ substituiert sind,
worin
R⁷ und R⁸ gleich oder verschieden sind und die oben angegebene Bedeutung von R¹ und R² haben,
X geradkettiges oder verzweigtes Alkyl oder Alkenyl mit jeweils bis zu 10 Kohlenstoffatomen bedeutet, die gegebenenfalls bis zu 2-fach durch Hydroxy oder Halogen substituiert sind,
R⁵ Wasserstoff bedeutet,
und
R⁶ Wasserstoff, Halogen, Mercapto, Azido, Trifluormethyl, Hydroxy, Trifluormethoxy, geradkettiges oder verzweigtes Alkoxy mit bis zu 5 Kohlenstoffatomen oder einen Rest der Formel -NR⁹R¹⁰ bedeutet,
worin
R⁹ und R¹⁰ gleich oder verschieden sind und die oben angegebene Bedeutung von R¹ und R² haben,
oder
R⁵ und R⁶ gemeinsam mit dem Kohlenstoffatom eine Carbonylgruppe bilden,
und deren Salze.

2. 2-Aryl-substituierte Pyridine der Formel nach Anspruch 1, in welcher
A und E gleich oder verschieden sind und für Phenyl oder Naphthyl stehen, die gegebenenfalls bis zu 2-fach gleich oder verschieden durch Fluor, Chlor, Brom, Hydroxy, Trifluormethyl, Trifluormethoxy, Nitro oder durch geradkettiges oder verzweigtes Alkyl, Acyl oder Alkoxy mit jeweils bis zu 6 Kohlenstoffatomen oder durch eine Gruppe der Formel -NR¹R² substituiert sind,
worin
R¹ und R² gleich oder verschieden sind und Wasserstoff, Phenyl oder geradkettiges oder verzweigtes Alkyl mit bis zu 4 Kohlenstoffatomen bedeuten,
D für geradkettiges oder verzweigtes Alkyl mit bis zu 7 Kohlenstoffatomen steht, das durch Hydroxy substituiert ist,
L für Cyclopropyl, Cyclobutyl, Cyclopentyl, Cyclohexyl oder Cycloheptyl steht, oder
für geradkettiges oder verzweigtes Alkyl mit bis zu 6 Kohlenstoffatomen steht, das gegebenenfalls durch Cyclopropyl, Cyclobutyl, Cyclopentyl, Cyclohexyl, Cycloheptyl oder durch Hydroxy substituiert ist,
T für einen Rest der Formel
R³―X―
oder 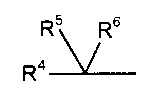 steht,
worin
R³ und R⁴ gleich oder verschieden sind und Cyclopropyl, Cyclopentyl oder Cyclohexyl bedeuten, oder Napthyl, Phenyl, Pyridyl, Chinolyl, Indolyl, Benzthiazolyl oder Tetrahydronaphthalinyl bedeuten, die gegebenenfalls bis zu 3-fach gleich oder verschieden durch Trifluormethyl, Trifluormethoxy, Fluor, Chlor, Brom, Hydroxy, Carboxyl, durch geradkettiges oder verzweigtes Alkyl, Acyl, Alkoxy oder Alkoxycarbonyl mit jeweils bis zu 5 Kohlenstoffatomen oder durch Phenyl, Phenoxy oder Phenylthio substituiert sind, die ihrerseits durch Fluor, Chlor, Brom, Trifluormethyl oder Trifluormethoxy substituiert sein können,
und/oder die Cyclen gegebenenfalls durch eine Gruppe der Formel -NR⁷R⁸ substituiert sind,
worin
R⁷ und R⁸ gleich oder verschieden sind und die oben angegebene Bedeutung von R¹ und R² haben,
X geradkettiges oder verzweigtes Alkyl oder Alkenyl mit jeweils bis zu 8 Kohlenstoffatomen bedeutet, die gegebenenfalls bis zu 2-fach durch Hydroxy oder Fluor substituiert sind,
R⁵ Wasserstoff bedeutet,
und
R⁶ Wasserstoff, Fluor, Chlor, Brom, Azido, Trifluormethyl, Hydroxy, Mercapto, Trifluormethoxy, geradkettiges oder verzweigtes Alkoxy mit bis zu 4 Kohlenstoffatomen oder einen Rest der Formel -NR⁹R¹⁰ bedeutet,
worin
R⁹ und R¹⁰ gleich oder verschieden sind und die oben angegebene Bedeutung von R¹ und R² haben,
oder
R⁵ und R⁶ gemeinsam mit dem Kohlenstoffatom eine Carbonylgruppe bilden,
und deren Salze.

3. 2-Aryl-substituierte Pyridine der Formel nach Anspruch 1, in welcher
A und E gleich oder verschieden sind und für Phenyl oder Naphthyl stehen, die gegebenenfalls bis zu 2-fach gleich oder verschieden durch Fluor, Chlor, Brom, Hydroxy, Trifluormethyl, Trifluormethoxy, Nitro oder durch geradkettiges oder verzweigtes Alkyl oder Alkoxy mit jeweils bis zu 5 Kohlenstoffatomen substituiert sind,
D für geradkettiges oder verzweigtes Alkyl mit bis zu 6 Kohlenstoffatomen steht, das durch Hydroxy substituiert ist,
L für Cyclopropyl, Cyclobutyl, Cyclopentyl, Cyclohexyl oder Cycloheptyl steht, oder für geradkettiges oder verzweigtes Alkyl mit bis zu 5 Kohlenstoffatomen steht, das gegebenenfalls durch Cyclopentyl oder Cyclohexyl substituiert ist,
T für einen Rest der Formel
R³―X―
oder 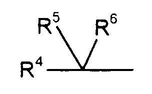 steht,
worin
R³ und R⁴ gleich oder verschieden sind und Cyclopropyl, Phenyl, Pyridyl, Chinolyl, Indolyl, Naphthyl, Benzthiazolyl oder Tetrahydronaphthalinyl bedeuten, die gegebenenfalls bis zu 2-fach gleich oder verschieden durch Trifluormethyl, Trifluormethoxy, Fluor, Chlor, Brom, Hydroxy, Carboxyl, Amino, durch geradkettiges oder verzweigtes Alkyl, Alkoxy oder Alkoxycarbonyl mit jeweils bis zu 4 Kohlenstoffatomen oder durch Phenyl, Phenoxy oder Phenylthio substituiert sind, die ihrerseits durch Fluor, Chlor, Brom, Trifluormethyl oder Trifluormethoxy substituiert sein können,
worin
X geradkettiges oder verzweigtes Alkyl oder Alkenyl mit bis zu 6 Kohlenstoffatomen bedeutet, die gegebenenfalls bis zu 2-fach durch Hydroxy oder Fluor substituiert sind,
R⁵ Wasserstoff bedeutet,
und
R⁶ Wasserstoff, Fluor, Chlor, Brom, Azido, Amino, Trifluormethyl, Hydroxy, Trifluormethoxy, Methoxy oder Mercapto bedeutet,
oder
R⁵ und R⁶ gemeinsam mit dem Kohlenstoffatom eine Carbonylgruppe bilden,
und deren Salze.

4. 2-Aryl-substituierte Pyridine der Formel nach Anspruch 1, in welcher
A für Phenyl steht, das gegebenenfalls bis zu 2-fach gleich oder verschieden durch Fluor, Chlor, Methyl oder Methoxy substituiert ist.

5. 2-Aryl-substituierte Pyridine nach Anspruch 1 bis 4 zur therapeutischen Anwendung.

6. Verfahren zur Herstellung von 2-Aryl-substituierten Pyridinen nach Anspruch 1 bis 4, dadurch gekennzeichnet, daß man Verbindungen der allgemeinen Formel (II) oder (III) 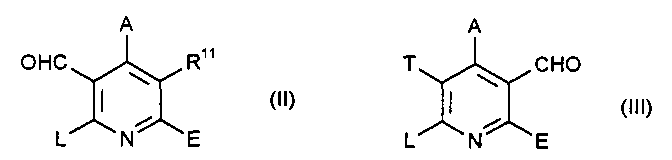 in welcher
A, E, L und T die oben angegebene Bedeutung haben,
und
R¹¹ für geradkettiges oder verzweigtes Alkoxycarbonyl mit bis zu 4 Kohlenstoffatomen steht,
entweder zunächst mit Grignard- oder Wittig-Reagenzien in inerten Lösemitteln umsetzt, gegebenenfalls weitere Derivatisierung nach üblichen Methoden durchführt und anschließend in inerten Lösemitteln reduziert,
oder im Fall der Verbindungen der allgemeinen Formel (III) direkt Reduktionen, gegebenenfalls über mehrere Stufen, durchführt.

7. Arzneimittel enthaltend 2-substituierte Pyridine nach Anspruch 1 bis 4 sowie ein pharamakologisch unbedenkliches Formulierungshilfsmittel.

8. Arzneimittel nach Anspruch 7 zur Behandlung von Lipoproteinaemia und Hyperlipoproteinaemia.

9. Verwendung von 2-Aryl-substituierten Pyridinen nach Anspruch 1 bis 4 zur Herstellung von Arzneimitteln.

10. Verwendung nach Anspruch 9 zur Herstellung von Arzneimitteln zur Behandlung von Lipoproteinaemia und Hyperlipoproteinaemia.

## Claims

1. 2-Aryl-substituted pyridines of the general formula (I) 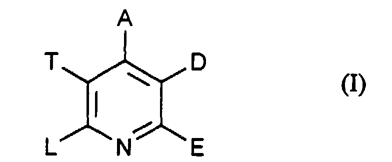 in which
A and E are identical or different and represent aryl having 6 to 10 carbon atoms, which is optionally substituted up to 5 times identically or differently by halogen, hydroxyl, trifluoromethyl, trifluoromethoxy, nitro or by straight-chain or branched alkyl, acyl, hydroxyalkyl or alkoxy each having up to 7 carbon atoms, or by a group of the formula -NR¹R²,
in which
R¹ and R² are identical or different and denote hydrogen, phenyl or straight-chain or branched alkyl having up to 6 carbon atoms,
D represents straight-chain or branched alkyl having up to 8 carbon atoms, which is substituted by hydroxyl,
L represents cycloalkyl having 3 to 8 carbon atoms or straight-chain or branched alkyl having up to 8 carbon atoms, which is optionally substituted by cycloalkyl having 3 to 8 carbon atoms or by hydroxyl,
T represents a radical of the formula
R³―X―
or 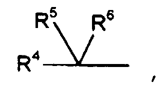 in which
R³ and R⁴ are identical or different and denote cycloalkyl having 3 to 8 carbon atoms, or
denote aryl having 6 to 10 carbon atoms, or a 5- to 7-membered aromatic, optionally benzo-fused heterocycle having up to 3 heteroatoms from the series S, N and/or O, each of which is optionally substituted up to 3 times identically or differently by trifluoromethyl, trifluoromethoxy, halogen, hydroxyl, carboxyl, nitro, by straight-chain or branched alkyl, acyl, alkoxy or alkoxycarbonyl each having up to 6 carbon atoms or by phenyl, phenoxy or phenylthio, which for their part can be substituted by halogen, trifluoromethyl or trifluoromethoxy,
and/or the cycles are optionally substituted by a group of the formula -NR⁷R⁸,
in which
R⁷ and R⁸ are identical or different and have the meaning of R¹ and R² indicated above,
X denotes straight-chain or branched alkyl or alkenyl each having up to 10 carbon atoms, each of which is optionally substituted up to 2 times by hydroxyl or halogen,
R⁵ denotes hydrogen
and
R⁶ denotes hydrogen, halogen, mercapto azido, trifluoromethyl, hydroxyl, trifluoromethoxy, straight-chain or branched alkoxy having up to 5 carbon atoms or a radical of the formula -NR⁹R¹⁰,
in which
R⁹ and R¹⁰ are identical or different and have the meaning of R¹ and R² indicated above,
or
R⁵ and R⁶ together with the carbon atom form a carbonyl group,
and their salts.

2. 2-Aryl-substituted pyridines of the formula according to Claim 1, in which
A and E are identical or different and represent phenyl or naphthyl, each of which is optionally substituted up to 2 times identically or differently by fluorine, chlorine, bromine, hydroxyl, trifluoromethyl, trifluoromethoxy, nitro or by straight-chain or branched alkyl, acyl or alkoxy each having up to 6 carbon atoms or by a group of the formula -NR¹R²,
in which
R¹ and R² are identical or different and denote hydrogen, phenyl or straight-chain or branched alkyl having up to 4 carbon atoms,
D represents straight-chain or branched alkyl having up to 7 carbon atoms, which is substituted by hydroxyl,
L represents cyclopropyl, cyclobutyl, cyclopentyl, cyclohexyl or cycloheptyl, or represents straight-chain or branched alkyl having up to 6 carbon atoms, which is optionally substituted by cyclopropyl, cyclobutyl, cyclopentyl, cyclohexyl, cycloheptyl or by hydroxyl,
T represents a radical of the formula
R³―X―
or 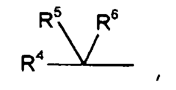 in which
R³ and R⁴ are identical or different and denote cyclopropyl, cyclopentyl or cyclohexyl, or denote naphthyl, phenyl, pyridyl, quinolyl, indolyl, benzothiazolyl or tetrahydronaphthalenyl, each of which is optionally substituted up to 3 times identically or differently by trifluoromethyl, trifluoromethoxy, fluorine, chlorine, bromine, hydroxyl, carboxyl, by straight-chain or branched alkyl, acyl, alkoxy or alkoxycarbonyl each having up to 5 carbon atoms or by phenyl, phenoxy or phenylthio, which for their part can be substituted by fluorine, chlorine, bromine, trifluoromethyl or trifluoromethoxy,
and/or the cycles are optionally substituted by a group of the formula -NR⁷R⁸,
in which
R⁷ and R⁸ are identical or different and have the meaning of
R¹ and R² indicated above,
X is straight-chain or branched alkyl or alkenyl each having up to 8 carbon atoms, each of which is optionally substituted up to 2 times by hydroxyl or fluorine,
R⁵ denotes hydrogen
and
R⁶ denotes hydrogen, fluorine, chlorine, bromine, azido, trifluoromethyl, hydroxyl, mercapto trifluoromethoxy, straight-chain or branched alkoxy having up to 4 carbon atoms or a radical of the formula -NR⁹R¹⁰,
in which
R⁹ and R¹⁰ are identical or different and have the meaning of
R¹ and R² indicated above,
or
R⁵ and R⁶ together with the carbon atom form a carbonyl group,
and their salts.

3. 2-Aryl-substituted pyridines of the formula according to Claim 1, in which
A and E are identical or different and represent phenyl or naphthyl, each of which is optionally substituted up to 2 times identically or differently by fluorine, chlorine, bromine, hydroxyl, trifluoromethyl, trifluoromethoxy, nitro or by straight-chain or branched alkyl or alkoxy each having up to 5 carbon atoms,
D represents straight-chain or branched alkyl having up to 6 carbon atoms, which is substituted by hydroxyl,
L represents cyclopropyl, cyclobutyl, cyclopentyl, cyclohexyl or cycloheptyl, or represents straight-chain or branched alkyl having up to 5 carbon atoms, which is optionally substituted by cyclopentyl or cyclohexyl,
T represents a radical of the formula
R³―X―
or 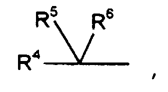 in which
R³ and R⁴ are identical or different and denote cyclopropyl, phenyl, pyridyl, quinolyl, indolyl, naphthyl, benzothiazolyl or tetrahydronaphthalenyl, each of which is optionally substituted up to 2 times identically or differently by trifluoromethyl, trifluoromethoxy, fluorine, chlorine, bromine, hydroxyl, carboxyl, amino, by straight-chain or branched alkyl, alkoxy or alkoxycarbonyl each having up to 4 carbon atoms or by phenyl, phenoxy or phenylthio, which for their part can be substituted by fluorine, chlorine, bromine, trifluoromethyl or trifluoromethoxy,
X denotes straight-chain or branched alkyl or alkenyl having up to 6 carbon atoms, each of which is optionally substituted up to 2 times by hydroxyl or fluorine,
R⁵ denotes hydrogen
and
R⁶ denotes hydrogen, fluorine, chlorine, bromine, azido, amino, trifluoromethyl, hydroxyl, trifluoromethoxy, methoxy or mercapto,
or
R⁵ and R⁶ together with the carbon atom form a carbonyl group,
and their salts.

4. 2-Aryl-substituted pyridines of the formula according to Claim 1, in which
A represents phenyl, which is optionally substituted up to 2 times identically or differently by fluorine, chlorine, methyl or methoxy.

5. 2-Aryl-substituted pyridines according to Claims 1 to 4 for therapeutic administration.

6. Process for the preparation of 2-aryl-substituted pyridines according to Claims 1 to 4, characterized in that compounds of the general formula (II) or (III) 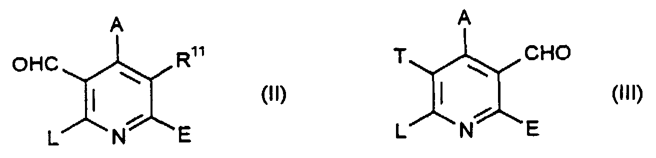 in which
A, E, L and T have the meaning indicated above
and
R¹¹ represents straight-chain or branched alkoxycarbonyl having up to 4 carbon atoms,
are either reacted first with Grignard or Wittig reagents, in inert solvents, further derivatization is optionally carried out according to customary methods and the products are then reduced in inert solvents, or in the case of the compounds of the general formula (III) direct reductions, optionally by means of several stages, are carried out.

7. Medicaments containing 2-substituted pyridines according to Claims 1 to 4 and a pharmacologically acceptable formulation auxiliary.

8. Medicaments according to Claim 7 for the treatment of lipoproteinaemia and hyperlipoproteinaemia.

9. Use of 2-aryl-substituted pyridines according to Claims 1 to 4 for the production of medicaments.

10. Use according to Claim 9 for the production of medicaments for the treatment of lipoproteinaemia and hyperlipoproteinaemia.

## Revendications

1. Pyridines portant un substituant aryle en position 2, répondant à la formule générale (I) 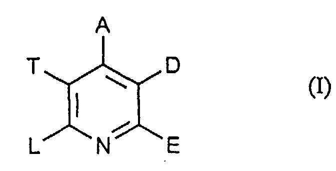 dans laquelle
A et E sont identiques ou différents et représentent un groupe aryle contenant de 6 à 10 atomes de carbone, qui porte le cas échéant jusqu'à 5 substituants identiques ou différents halogéno, hydroxyle, trifluorométhyle, trifluorométhoxy, nitro ou jusqu'à 5 substituants identiques ou différents alkyle à chaîne droite ou ramifiée, acyle, hydroxyalkyle ou alcoxy contenant respectivement jusqu'à 7 atomes de carbone, ou encore, à titre de substituant, un groupe répondant à la formule -NR¹R²
dans laquelle
R¹ et R² sont identiques ou différents et représentent un atome d'hydrogène, un groupe phényle ou un groupe alkyle à chaîne droite ou ramifiée contenant jusqu'à 6 atomes de carbone,
D représente un groupe alkyle à chaîne droite ou ramifiée contenant jusqu'à 8 atomes de carbone, qui porte un ou plusieurs substituants hydroxyle,
L représente un groupe cycloalkyle contenant de 3 à 8 atomes de carbone ou représente un groupe alkyle à chaîne droite ou ramifiée contenant jusqu'à 8 atomes de carbone, qui porte le cas échéant un ou plusieurs substituants cycloalkyle contenant de 3 à 8 atomes de carbone ou un ou plusieurs substituants hydroxyle,
T représente un radical répondant aux formules
R³―X―
ou 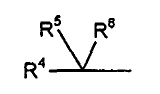 dans lesquelles
R³ et R⁴ sont identiques ou différents et représentent un groupe cycloalkyle contenant de 3 à 8 atomes de carbone,
ou représentent un groupe aryle contenant de 6 à 10 atomes de carbone, ou encore un composé hétérocyclique aromatique penta- à heptagonal le cas échéant benzocondensé contenant jusqu'à 3 hétéroatomes choisis parmi la série comprenant un atome de soufre, un atome d'azote et/ou un atome d'oxygène qui portent le cas échéant jusqu'à 3 substituants identiques ou différents trifluorométhyle, trifluorométhoxy, halogéno, hydroxyle, carboxyle, nitro, jusqu'à 3 substituants identiques
ou différents alkyle à chaîne droite ou ramifiée, acyle, alcoxy ou alcoxycarbonyle contenant respectivement jusqu'à 6 atomes de carbone ou jusqu'à 3 substituants identiques ou différents phényle, phénoxy ou phénylthio qui, quant à eux, peuvent porter un ou plusieurs substituants halogéno, trifluorométhyle ou trifluorométhoxy,
et/ou les cycles portent, le cas échéant, à titre de substituants, un groupe répondant à la formule -NR⁷R⁸ dans laquelle
R⁷ et R⁸ sont identiques ou différents et possèdent la signification de R¹ et de R² indiquée ci-dessus,
X représente un groupe alkyle ou un groupe alcényle à chaîne droite ou ramifiée contenant respectivement jusqu'à 10 atomes de carbone qui portent le cas échéant jusqu'à 2 substituants hydroxyle ou halogéno,
R⁵ représente un atome d'hydrogène
et
R⁶ représente un atome d'hydrogène, un atome d'halogène, un groupe mercapto, un groupe azido, un groupe trifluorométhyle, un groupe hydroxyle, un groupe trifluorométhoxy, un groupe alcoxy à chaîne droite ou ramifiée contenant jusqu'à 5 atomes de carbone, ou encore un radical répondant à la formule -NR⁹R¹⁰
dans laquelle
R⁹ et R¹⁰ sont identiques ou différents et possèdent la signification de R¹ et de R² indiquée ci-dessus
ou
R⁵ et R⁶ forment ensemble avec l'atome de carbone un groupe carbonyle,
ainsi que leurs sels.

2. Pyridines portant un substituant aryle en position 2, répondant à la formule selon la revendication 1, dans lesquelles
A et E sont identiques ou différents et représentent un groupe phényle ou un groupe naphtyle qui porte le cas échéant jusqu'à 2 substituants identiques ou différents fluoro, chloro, bromo, hydroxyle, trifluorométhyle, trifluorométhoxy, nitro, ou jusqu'à 2 substituants identiques ou différents alkyle à chaîne droite ou ramifiée, acyle ou alcoxy contenant respectivement jusqu'à 6 atomes de carbone, ou encore, à titre de substituant, un groupe répondant à la formule -NR¹R²
dans laquelle
R¹ et R² sont identiques ou différents et représentent un atome d'hydrogène, un groupe phényle ou un groupe alkyle à chaîne droite ou ramifiée contenant jusqu'à 4 atomes de carbone,
D représente un groupe alkyle à chaîne droite ou ramifiée contenant jusqu'à 7 atomes de carbone, qui porte un ou plusieurs substituants hydroxyle,
L représente un groupe cyclopropyle, un groupe cyclobutyle, un groupe cyclopentyle, un groupe cyclohexyle ou un groupe cycloheptyle, ou représente un groupe alkyle à chaîne droite ou ramifiée contenant jusqu'à 6 atomes de carbone, qui porte le cas échéant un ou plusieurs substituants cyclopropyle, cyclobutyle, cyclopentyle, cyclohexyle, cycloheptyle ou un ou plusieurs substituants hydroxyle,
T représente un radical répondant aux formules
R³―X―
ou 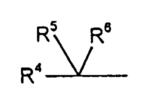 dans lesquelles
R³ et R⁴ sont identiques ou différents et représentent un groupe cyclopropyle, un groupe cyclopentyle ou un groupe cyclohexyle, ou représentent un groupe naphtyle, un groupe phényle, un groupe pyridyle, un groupe quinolyle, un groupe indolyle, un groupe benzthiazolyle ou un groupe tétrahydronaphtalinyle qui portent le cas échéant jusqu'à 3 substituants identiques ou différents trifluorométhyle, trifluorométhoxy, fluoro, chloro, bromo, hydroxyle, carboxyle, jusqu'à 3 substituants identiques ou différents alkyle à chaînes droites ou ramifiées, acyle, alcoxy ou alcoxycarbonyle contenant respectivement jusqu'à 5 atomes de carbone, ou jusqu'à 3 substituants identiques ou différents phényle, phénoxy ou phénylthio qui, quant à eux, peuvent porter un ou plusieurs substituants fluoro, chloro, bromo, trifluorométhyle ou trifluorométhoxy,
et/ou les cycles portent, le cas échéant, à titre de substituants, un groupe répondant à la formule -NR⁷R⁸
dans laquelle
R⁷ et R⁸ sont identiques ou différents et possèdent la signification de R¹ et de R² indiquée ci-dessus,
X représente un groupe alkyle ou un groupe alcényle à chaîne droite ou ramifiée contenant respectivement jusqu'à 8 atomes de carbone qui portent le cas échéant jusqu'à 2 substituants hydroxyle ou fluoro,
R⁵ représente un atome d'hydrogène
et
R⁶ représente un atome d'hydrogène, un atome de fluor, un atome de chlore, un atome de brome, un groupe azido, un groupe trifluorométhyle, un groupe hydroxyle, un groupe mercapto, un groupe trifluorométhoxy, un groupe alcoxy à chaîne droite ou ramifiée contenant jusqu'à 4 atomes de carbone ou encore un radical répondant à la formule -NR⁹R¹⁰
dans laquelle
R⁹ et R¹⁰ sont identiques ou différents et possèdent la signification de R¹ et de R² indiquée ci-dessus
ou
R⁵ et R⁶ forment ensemble avec l'atome de carbone un groupe carbonyle,
ainsi que leurs sels.

3. Pyridines portant un substituant aryle en position 2 selon la revendication 1, dans lesquelles
A et E sont identiques ou différents et représentent un groupe phényle ou un groupe naphtyle qui porte le cas échéant jusqu'à 2 substituants identiques ou différents fluoro, chloro, bromo, hydroxyle, trifluorométhyle, trifluorométhoxy, nitro ou jusqu'à 2 substituants identiques ou différents alkyle ou alcoxy à chaîne droite ou ramifiée contenant respectivement jusqu'à 5 atomes de carbone,
D représente un groupe alkyle à chaîne droite ou ramifiée contenant jusqu'à 6 atomes de carbone, qui porte un ou plusieurs substituants hydroxyle,
L représente un groupe cyclopropyle, un groupe cyclobutyle, un groupe cyclopentyle, un groupe cyclohexyle ou un groupe cycloheptyle, ou représente un groupe alkyle à chaîne droite ou ramifiée contenant jusqu'à 5 atomes de carbone, qui porte le cas échéant un ou plusieurs substituants cyclopentyle ou cyclohexyle,
T représente un radical répondant aux formules
R³―X―
ou 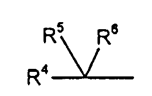 dans lesquelles
R³ et R⁴ sont identiques ou différents et représentent un groupe cyclopropyle, un groupe phényle, un groupe pyridyle, un groupe quinolyle, un groupe indolyle, un groupe naphtyle, un groupe benzthiazolyle ou un groupe tétrahydronaphtalinyle qui portent le cas échéant jusqu'à 2 substituants identiques ou différents trifluorométhyle, trifluorométhoxy, fluoro, chloro, bromo, hydroxyle, carboxyle, amino, jusqu'à 2 substituants identiques ou différents alkyle à chaînes droites ou ramifiées, alcoxy ou alcoxycarbonyle contenant respectivement jusqu'à 4 atomes de carbone, ou jusqu'à 2 substituants identiques ou différents phényle, phénoxy ou phénylthio qui, quant à eux, peuvent porter un ou plusieurs substituants fluoro, chloro, bromo, trifluorométhyle ou trifluorométhoxy,
X représente un groupe alkyle ou un groupe alcényle à chaîne droite ou ramifiée contenant jusqu'à 6 atomes de carbone qui portent le cas échéant jusqu'à 2 substituants hydroxyle ou fluoro,
R⁵ représente un atome d'hydrogène
et
R⁶ représente un atome d'hydrogène, un atome de fluor, un atome de chlore, un atome de brome, un groupe azido, un groupe amino, un groupe trifluorométhyle, un groupe hydroxyle, un groupe trifluorométhoxy, un groupe méthoxy ou un groupe mercapto
ou
R⁵ et R⁶ forment ensemble avec l'atome de carbone un groupe carbonyle,
ainsi que leurs sels.

4. Pyridines portant un substituant aryle en position 2 répondant à la formule selon la revendication 1, dans lesquelles
A représente un groupe phényle qui porte le cas échéant jusqu'à 2 substituants identiques ou différents fluoro, chloro, méthyle ou méthoxy.

5. Pyridines portant un substituant aryle en position 2 selon les revendications 1 à 4 pour l'application thérapeutique.

6. Procédé pour la préparation de pyridines portant un substituant aryle en position 2 selon les revendications 1 à 4, caractérisé en ce que, soit on fait d'abord réagir des composés répondant aux formules générales (II) ou (III) 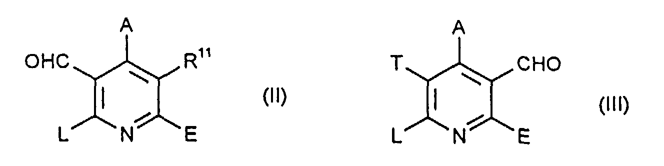 dans lesquelles
A, E, L et T ont la signification indiquée ci-dessus
et
R¹¹ représente un groupe alcoxycarbonyle à chaîne droite ou ramifiée contenant jusqu'à 4 atomes de carbone,
avec des réactifs de Grignard ou de Wittig dans des solvants inertes; on procède le cas échéant à une dérivation ultérieure conformément à des procédés habituels; ensuite, on réduit dans des solvants inertes,
soit, dans le cas des composés répondant à la formule générale (III), on effectue directement des réductions, le cas échéant en passant par plusieurs étapes.

7. Médicament contenant des pyridines substituées en position 2 selon les revendications 1 à 4, ainsi qu'un adjuvant de formulation acceptable du point de vue pharmacologique.

8. Médicament selon la revendication 7 pour le traitement de la lipoprotéinémie et de l'hyperlipoprotéinémie.

9. Utilisation de pyridines portant un substituant aryle en position 2 selon les revendications 1 à 4 pour la préparation de médicaments.

10. Utilisation selon la revendication 9 pour la préparation de médicaments pour le traitement de la lipoprotéinémie et de l'hyperlipoprotéinémie.
